(19) Europäisches Patentamt
European Patent Office
Office européen des brevets

(11) EP 2 928 861 B1

(12) **FASCICULE DE BREVET EUROPEEN**

(45) Date de publication et mention de la délivrance du brevet:
**11.01.2017 Bulletin 2017/02**

(21) Numéro de dépôt: **13811402.0**

(22) Date de dépôt: **04.12.2013**

(51) Int Cl.:
***C07C 235/40*** *(2006.01)* ***C07C 237/24*** *(2006.01)*
***C07D 307/00*** *(2006.01)* ***A61K 31/165*** *(2006.01)*
***A61P 25/24*** *(2006.01)*

(86) Numéro de dépôt international:
**PCT/EP2013/075481**

(87) Numéro de publication internationale:
**WO 2014/086825 (12.06.2014 Gazette 2014/24)**

(54) **DÉRIVÉS D'AMINOCYCLOBUTANE, LEUR PROCÉDÉ DE PRÉPARATION ET LEUR UTILISATION À TITRE DE MÉDICAMENTS**

AMINOCYCLOBUTANDERIVATE, VERFAHREN ZUR HERSTELLUNG DAVON UND VERWENDUNG DAVON ALS ARZNEIMITTEL

AMINOCYCLOBUTANE DERIVATIVES, METHOD FOR PREPARING SAME AND THE USE THEREOF AS DRUGS

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **04.12.2012 FR 1261621**

(43) Date de publication de la demande:
**14.10.2015 Bulletin 2015/42**

(73) Titulaire: **Pierre Fabre Médicament**
**92100 Boulogne-Billancourt (FR)**

(72) Inventeurs:
- **VACHER, Bernard**
**81100 Castres (FR)**
- **BLANC, Elodie**
**81570 Semalens (FR)**
- **DEPOORTERE, Ronan**
**81100 Castres (FR)**

(74) Mandataire: **Regimbeau**
**20, rue de Chazelles**
**75847 Paris Cedex 17 (FR)**

(56) Documents cités:
**EP-A1- 0 747 348** **WO-A1-2010/112597**
**WO-A2-03/063797** **US-A1- 2008 268 071**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).



Printed by Jouve, 75001 PARIS (FR)

## Description

La présente invention concerne des dérivés d'aminocyclobutane, ainsi que leur procédé de préparation et leur utilisation en thérapeutique humaine.

Les récepteurs au glutamate du sous-type NMDA (acide N-méthyl-D-aspartique) sont des récepteurs ionotropes, principalement perméables aux ions $Ca^{++}$. Physiologiquement, leur activation déclenche l'ouverture d'un canal ionique et la production d'un courant entrant qui ne s'inactive que lentement. La stimulation de ce récepteur nécessite la présence simultanée de glutamate (agoniste endogène) et de glycine ou de D-sérine (co-agonistes endogènes) ainsi qu'une dépolarisation de la membrane plasmique initiée par des courants non-NMDA. Les récepteurs NMDA sont abondants au niveau du système nerveux central et sont également présents en périphérie. On les trouve au niveau des neurones, des astrocytes et des oligodendrocytes (Karadottir et al., 2005, Nature, 438, 1162-1166). Au niveau neuronal, ils sont localisés majoritairement au niveau post-synaptique, mais aussi dans les régions extra-synaptiques le long des axones. Les récepteurs NMDA jouent un rôle clef dans la communication et la plasticité neuronale, ainsi que dans l'exitotoxicité.

L'activité physiologique des récepteurs NMDA est essentielle pour un fonctionnement neuronal normal (Chen et Lipton, 2006, J. Neurochem., 97, 1611-1626). Par contre, la sur-activation de ces récepteurs est impliquée aussi bien dans les atteintes neuronales aiguës, par exemple un accident vasculaire cérébral ou un traumatisme crânien, que dans des conditions de stress chroniques, par exemple des maladies neurodégénératives. Elle est aussi l'une des principales causes de l'hyperexcitabilité qui conduit aux crises d'épilepsie. Les pathologies considérées comme associées à une hyperactivité des récepteurs NMDA, donc potentiellement sensibles aux antagonistes NMDA, sont nombreuses. On peut citer à titre d'exemple : l'épilepsie, les maladies neurodégénératives comme la maladie de Huntington, la maladie de Parkinson, la maladie d'Alzheimer, les accidents vasculaires cérébraux, la sclérose latérale amyotrophique ou en plaque, la démence du sida, l'anxiété, la dépression et les syndromes douloureux.

Dans la présente invention, la Demanderesse s'intéresse tout particulièrement aux propriétés antidépressives et analgésiques des antagonistes des récepteurs NMDA de formule (1).

L'expression douleur chronique, utilisée dans le cadre de la présente demande de brevet, regroupe les syndromes douloureux dont l'évolution dure plus de trois mois, mais dont l'intensité peut varier au cours du temps. Au contraire, le terme douleur aiguë qualifie des douleurs qui durent moins de trois mois.

Dans le cadre de la présente invention, la douleur est définie comme une expérience sensorielle et émotionnelle anormale, désagréable, voire pénible, qui est perçue et intégrée au niveau le plus élevé du cortex cérébral, ce qui lui confère sa tonalité émotionnelle et affective. Par "analgésie", on entend une diminution de l'intensité de la douleur ressentie en réponse à un stimulus douloureux. Par "médicaments analgésiques" (ou "analgésiques"), on entend des médicaments qui soulagent ou suppriment la douleur sans entraîner de perte de sensations ou de la conscience.

Des douleurs d'étiologies différentes nécessitent des stratégies thérapeutiques différentes. On distingue en général plusieurs types de douleurs classées selon les mécanismes mis en clause :

- les douleurs par excès de nociception résultent de lésions ou d'excitations (par exemple inflammation) des tissus périphériques ou viscéraux ;
- les douleurs neuropathiques (ou neurogènes) sont liées à une lésion ou un dysfonctionnement ou une perturbation du système somatosensoriel ; elles se distinguent des douleurs nociceptives par une sémiologie différente ;
- les douleurs psychogènes (ou idiopathiques) sont des douleurs qui existent en l'absence de lésions. Les mécanismes physiologiques de ces douleurs ne sont pas clairement définis. Elles sont généralement résistantes aux analgésiques.

Certaines douleurs ont, toutefois, des caractéristiques communes à plusieurs types de douleurs. C'est par exemple le cas des douleurs du bas du dos ou cancéreuses qui se présentent sous forme de douleurs par excès de nociception ou neuropathiques ou le plus souvent mixtes.

La dépression est définie en psychiatrie comme un trouble de l'humeur. Elle se caractérise par une perte de motivation associée ou non à différents symptômes, tels qu'une perte d'espoir, d'estime de soi, de l'anxiété, de l'angoisse et, dans certains cas extrêmes, des hallucinations. Elle est souvent multifactorielle et ses causes généralement multiples.

Il est rapporté qu'environ 7 % des Européens en sont atteints et qu'un tiers d'entre eux sont résistants aux antidépresseurs utilisés en clinique. Le coût de la dépression pour la société chez les 15-44 ans est parmi le plus élevé de toutes les pathologies connues. Un objectif de la présente invention est précisément de décrire des nouveaux antagonistes NMDA qui présentent des propriétés avantageuses dans cette indication pour laquelle les traitements existants ne sont pas entièrement satisfaisants.

Il a été montré chez la souris que l'administration chronique d'antidépresseurs de mécanismes d'action différents : inhibiteurs de monoamine oxydase, tricycliques, inhibiteurs de capture de la sérotonine (SSRI), ou inhibiteurs mixtes de la sérotonine et de la noradrénaline (SNRI), modifiait la distribution et la densité des récepteurs NMDA. Chez

le rat, une administration aiguë par voie intrapéritonéale de kétamine, un antagoniste des récepteurs NMDA, diminue le temps d'immobilité dans le test de la nage forcée, un modèle préclinique reconnu pour la mise en évidence de l'activité antidépressive de molécules. De plus, des études récentes indiquent que la kétamine possède des propriétés antidépressives chez l'homme. Ainsi, l'administration d'une dose unique sub-anesthésique de kétamine par voie intraveineuse chez des patients atteints de dépression résistante améliore de façon significative leur état, et ce 2 h à peine après injection. Les effets antidépresseurs obtenus sont, de plus, maintenus pendant plus d'une semaine (Zarate et al., 2006, Arch. Gen. Psychiatry, 63, 856-864). Cette rapidité d'action contraste avec le délai d'action observé avec les antidépresseurs classiques, c'est-à-dire tricycliques de première génération, SSRI ou SNRI, qui nécessitent plusieurs semaines de traitement avant de montrer un éventuel bénéfice. Il apparaît donc que des antagonistes du récepteur NMDA, et en particulier la kétamine, soient efficaces dans le traitement de la dépression et notamment dans le traitement des dépressions résistantes aux médicaments existants.

Les besoins thérapeutiques dans le traitement de la douleur sont considérables. En effet, un nombre incalculable d'individus souffre de douleurs aiguës, et plus d'un adulte sur cinq, en Europe comme aux Etats-Unis, souffre de douleurs chroniques (Johannes et al., 2010, J. Pain, 11, 1230-1239). La présente invention a pour objet de décrire les propriétés analgésiques avantageuses dont sont dotés les composés de formule (1) et les perspectives thérapeutiques qu'ils ouvrent dans le domaine du traitement des douleurs aiguës et chroniques.

De nombreuses études chez l'animal et l'homme ont montré que les antagonistes des récepteurs NMDA tels que la kétamine pouvaient soulager des douleurs d'étiologies multiples comme, par exemple, les douleurs neuropathiques, postopératoires ou cancéreuses (Cohen et al., 2011, Adv. Psychosom. Med., 30, 139-161). Ainsi, la kétamine par voie intraveineuse réduit les douleurs neuropathiques chez des patients résistants aux traitements par les antidépresseurs conventionnels. Elle améliore également l'allodynie et l'hyperalgie chez les patients atteints de CRPS *(complex régional pain syndrome*) (Finch et al., 2009, Pain, 146, 18-25). Comme adjuvant, la kétamine administrée à faible dose en péri-opératoire diminue la consommation d'analgésiques et limite la tolérance aiguë à la morphine dans les suites opératoires (Elia et Tramer, 2005, Pain, 113, 61-70). En traitement préventif, la kétamine et le dextrométhorphane (un autre antagoniste NMDA) améliorent la prise en charge de la douleur postopératoire (Muir, 2006, Current Opinion in Pharmacology, 6, 53-60). La kétamine semble également prévenir la survenue de douleurs chroniques post-chirurgicales (Wilder-Smith et al., 2002, Pain, 97, 189-194). Les résultats obtenus avec d'autres antagonistes NMDA tels que l'amantadine ou le MK-801 dans les douleurs neuropathiques n'ont cependant pas été concluants (Muir, 2006, déjà cité).

L'ouverture des canaux NMDA provoque une augmentation du calcium intracellulaire qui active, entre autres, la NO synthétase et la cyclooxygénase de type II à l'origine de la synthèse des prostaglandines (PGs). Les antagonistes NMDA au travers de l'inhibition des PGs, notamment de la PGE2, ont donc une action directe sur la régulation de l'état inflammatoire (Beloeil et al., 2009, Anesth. Analg., 109, 943-950). Cette activité anti-inflammatoire complémentaire des antagonistes NMDA peut être avantageuse pour le traitement des douleurs aiguës ou chroniques d'origine inflammatoire. De même, les récepteurs NMDA sont exprimés dans les chondrocytes et participent à la fonction mécanique de ces cellules (Salter et al., 2004, Biorheology, 41, 273-281). Ils semblent notamment impliqués dans leur prolifération et dans l'inflammation conduisant à la destruction du cartilage articulaire (Piepoli et al., 2009, Osteoarthritis and Cartilage, 17, 1076-1083). Comme ce dernier ne se régénère pas chez l'adulte, l'utilisation d'un antagoniste NMDA semble donc particulièrement avantageuse pour prévenir ou ralentir la destruction du cartilage articulaire survenant dans les pathologies telles que, par exemple, les monoarthrites inflammatoires, l'arthrite rhumatoïde, l'arthrite septique, l'ostéoarthrite, la polyarthrite rhumatoïde, la goutte, la spondylarthrite, les rhumatismes abarticulaires aigus.

Toutefois, l'utilité des antagonistes NMDA en clinique humaine est limitée par leurs effets indésirables, notamment au niveau du système nerveux central et en particulier lors de traitements répétés. Parmi les effets secondaires des antagonistes NMDA, on peut citer, par exemple : les hallucinations, la confusion, les troubles de la personnalité, les cauchemars, l'agitation, le défaut de concentration, les altérations de l'humeur, les convulsions, la sédation, la somnolence, les nausées (Aarts et Tymianski, 2003, Biochem. Pharmacol., 66, 877-886). Ces effets secondaires résultent du fait que les antagonistes NMDA ne bloquent pas seulement l'activation excessive du système glutamate/NMDA, mais qu'ils perturbent également sa fonction physiologique normale. En pratique, il apparaît donc indispensable d'améliorer le rapport bénéfice/risque des antagonistes NMDA disponibles en clinique.

Lorsque le type de douleur à traiter s'y prête, comme par exemple dans le cas d'arthrites, le rapport bénéfice/risque de l'antagoniste NMDA peut être amélioré en limitant son action sur le système nerveux central, par exemple au moyen d'une application topique. La concentration du composé au niveau du tissu cible est alors très supérieure à sa concentration dans le sang réduisant ainsi les risques de toxicité. Plusieurs antagonistes NMDA ont ainsi été étudiés par voie épidurale ou topique. Ainsi, la kétamine en application locale a montré une efficacité dans le traitement des douleurs neuropathiques non soulagées par les médicaments classiques. Différentes associations entre un antagoniste NMDA et un ou plusieurs autres agents analgésiques ont aussi été étudiées en application locale. Par exemple, la kétamine ou d'autres antagonistes NMDA ont été combinés avec des antidépresseurs ou des antihypertenseurs (US 6 387 957) ; des antiépileptiques (WO 03/061656, WO 98/07447, WO 99/12537, US 20040204366, WO 2010036937) ;

des agonistes adrénergiques (US 20040101582) ; ou des opioïdes (WO 2000003716).

Compte tenu du rôle essentiel joué par les récepteurs NMDA dans nombre de pathologies psychiatriques et neurologiques, ils ont fait l'objet d'une recherche intensive et une multitude d'antagonistes/bloqueurs/modulateurs ont été décrits. Schématiquement, ils peuvent être classés en trois grandes familles selon leur site d'action sur le récepteur NMDA. On distingue ainsi :

1) les antagonistes compétitifs qui ciblent les sites de liaison soit du glutamate, par exemple le selfotel, le perzinfotel et les prodrogues (WO 2009029618) ; soit de la glycine, par exemple le gavestinel, GV-196771 (Wallace et al., 2002, Neurology, 59, 1694-1700) et les quinolines rapportées dans la demande de brevet WO 2010037533. On inclut dans cette catégorie les agonistes partiels du site glycine tels que la D-cyclosérine (US 2011160260).

2) les antagonistes non-compétitifs (ou allostériques) qui agissent au niveau des nombreux sites modulateurs de la régulation du récepteur, tels que par exemple les sites des polyamines, des phenyléthanolamines. Les composés appartenant à cette famille sont actuellement les plus étudiés en clinique. Un des chefs de file est l'ifenprodil (23210-56-2) et des dérivés plus sélectifs que ce dernier vis-à-vis du récepteur NMDA sont en cours d'évaluation clinique comme, par exemple, le traxodopril, le RGH-896, le MK-0657, EVT-101 et EVT-103 (Mony et al., 2009, Br. J. Pharmacol., 157, 1301-1317).

3) les antagonistes non-compétitifs, bloqueurs du pore du canal. C'est la famille qui a eu le plus de succès en clinique puisque la kétamine (Ketalar®, anesthétique/analgésique), le dextrométhorphane (Atuxane®, antitussif), la mémantine (Ebixa®, anti-Alzheimer), l'amantadine (Mantadix®, antiviral puis antiparkinsonien), le felbamate (Taloxa®, anticonvulsivant) sont commercialisés. La phéncyclidine (Sernyl®) développée comme anesthésique a été retirée du marché et la dizocilpine (MK-801) n'est pas commercialisée en tant que médicament.

Les composés de l'invention appartiennent à cette dernière famille des antagonistes non-compétitifs, bloqueurs du canal du récepteur NMDA. Un avantage majeur des composés de ce type réside dans le fait qu'ils ne bloquent le canal que lorsque ce dernier est ouvert, et sont de ce fait d'autant plus efficaces que l'activité du récepteur NMDA est excessive. On conçoit aussi facilement que les caractéristiques biophysiques du bloqueur/antagoniste, qui vont conditionner la fréquence et la durée d'ouverture du canal, vont jouer un rôle déterminant sur son activité pharmacologique et son rapport bénéfice/risque. Plusieurs composés de ce type sont en études cliniques comme, par exemple, le CNS-5161 (160754-76-7), le néramexane (219810-59-0), le dimiracétam (126100-97-8), V-3381 (1104525-45-2), NEU-2000 (640290-67-1). D'autres sont au stade préclinique, on peut citer, à titre d'exemple, les oxazolidines revendiquées dans la demande de brevet WO 2009092324, les indanes (WO 2009069610), les diaryléthylamines (WO 2010074647), les arylcyclohexylamines (WO 2010142890), les analogues de la kétamine et de la phéncyclidine (Zarantonello et al., 2011, Bioorg. Med. Chem. Lett., 21, 2059-2063).

L'illustration de l'état de la technique antérieure peut être complétée par la demande EP 0 747 348 qui décrit des dérivés du 1-phényl-2-aminoalkyl-cyclopropylecarboxamide comme antagonistes des récepteurs NMDA.

La présente invention concerne les dérivés représentés par la formule générale (1) :

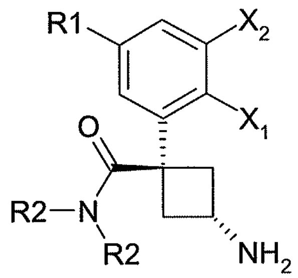

(1)

dans laquelle :

- $X_1$ représente un atome d'hydrogène ou un atome de fluor ;
- $X_2$ est un atome d'hydrogène ou un atome de fluor ou un atome de chlore ;
- R1 représente un atome d'hydrogène ou un atome de fluor ou un atome de chlore ou un groupe méthyle ou un groupe méthoxy ou un groupe cyano ;
- R2 représente ensemble ou séparément un groupe méthyle ou un groupe éthyle.

Préférentiellement, les composés de formule générale (1) selon l'invention sont ceux dans lesquels :

- $X_1$ représente un atome d'hydrogène ou un atome de fluor ;

- $X_2$ est un atome d'hydrogène ou un atome de fluor ou un atome de chlore ;
- R1 représente un atome d'hydrogène ou un atome de fluor ou un atome de chlore ou un groupe méthyle ou un groupe méthoxy ou un groupe cyano ;
- R2 est un groupe éthyle.

Les composés de l'invention peuvent intervenir en tant que diastéréoisomères purs ou mélanges de diastéréoisomères. Plus spécifiquement, l'invention se rapporte aux diastéréoisomères purs dans lequel le groupe 1-carboxamide et le groupe 3-amino occupent les faces opposées du plan défini par le cyclobutane. Cette relation stéréochimique entre lesdits substituants est qualifiée de 'trans' dans la présente invention. L'invention se rapporte donc aux diastéréoisomères trans purs des produits suivants :

- *trans*-3-amino-*N,N*-diéthyl-1-phénylcyclobutanecarboxamide,
- *trans*-3-amino-*N,N*-diméthyl-1-phénylcyclobutanecarboxamide
- *trans*-3-amino-*N,N*-diéthyl-1-(2-fluorophényl)-cyclobutanecarboxamide,
- *trans*-3-amino-*N,N*-diéthyl-1-(3-méthoxyphényl)-cyclobutanecarboxamide,
- *trans*-3-amino-*N,N*-diéthyl-1-(3-fluorophényl)-cyclobutanecarboxamide,
- *trans*-3-amino-*N,N*-diéthyl-1-(3-chlorophényl)-cyclobutanecarboxamide,
- *trans*-3-amino-*N,N*-diéthyl-1-(3-méthylphényl)-cyclobutanecarboxamide,
- *trans*-3-amino-*N,N*-diéthyl-1-(3-cyanophényl)-cyclobutanecarboxamide,
- *trans*-3-amino-*N,N*-diéthyl-1-(2-fluoro-3-chlorophényl)-cyclobutanecarboxamide,
- *trans*-3-amino-*N,N*-diéthyl-1-(2,5-difluorophényl)-cyclobutanecarboxamide,
- *trans*-3-amino-*N,N*-diéthyl-1-(3,5-difluorophényl)-cyclobutanecarboxamide,
- *trans*-3-amino-*N,N*-diéthyl-1-(3,5-dichlorophényl)-cyclobutanecarboxamide
  ainsi qu'à leurs sels pharmaceutiquement acceptables.

Par "diastéréoisomères purs", il est entendu que le diastéréoisomère 'trans' du composé de formule générale (1) contient moins de 5 % du diastéréoisomère 'cis", c'est-à-dire celui dans lequel le groupe 1-carboxamide et le groupe 3-amino occupent le même demi-espace du plan défini par le cyclobutane.
Par "diastéréoisomères", on entend au sens de la présente invention des stéréoisomères qui ne sont pas des images l'un de l'autre dans un miroir.
Par "stéréoisomères", on entend au sens de la présente invention des isomères de constitution identique mais qui diffèrent par l'arrangement de leurs atomes dans l'espace.
L'état de la technique le plus proche est représenté par les dérivés décrit dans la demande de brevet WO 2003063797 et répondant à la formule suivants :

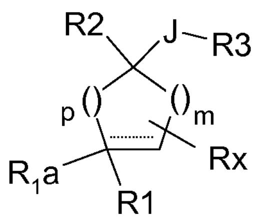

dans laquelle :

m et p sont indépendamment égale à 0, 1, 2 ou 3 ;
la liaison en pointillés représente éventuellement une double liaison lorsque le groupe $R_1a$ est absent ;
R1 peut être un groupe $NR_6R_7$ avec $R_6$ et $R_7$ représentant éventuellement un atome d'hydrogène ;
$R_1a$ peut être un atome d'hydrogène ;
R2 peut être un groupe aryle substitué ou non ;
J peut être une liaison ;
R3 peut être un groupe -C($Z_1$)-$R_5$ avec $R_5$ représentant éventuellement un groupe $NR_6aR_7a$ ;
$R_6a$ et $R_7a$ représentent éventuellement un groupe alkyle substitué ou non et $Z_1$ représentant éventuellement un groupe carbonyle (C=O) ;
Rx peut être un ou plusieurs groupes substitué (s) ou non attaché(s) à tous les atomes de carbone du cycle disponible mais aussi un atome d'hydrogène.

Cette demande de brevet couvre donc un nombre considérable de composés dont un grand nombre de dérivés

du type cyclobutane (m = 0 et p = 1). Parmi ces derniers, seuls quatre sont exemplifiés dans ledit brevet. Il s'agit des composés de formule suivante :

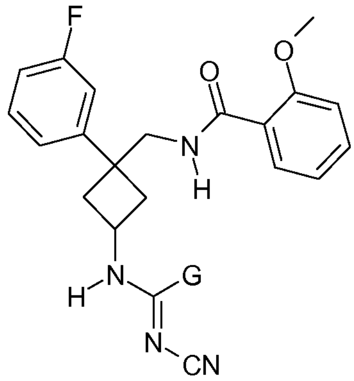

dans laquelle G est un groupe $NH_2$ ou $N(CH_3)2$ ou $NH(CH_2CH_3)$ ou $NH(CH_2CHCH_2)$.

Les composés de cette demande de brevet sont revendiqués comme étant des inhibiteurs du courant produit par les canaux potassiques voltage dépendants du type Kv1 et en particulier celui produit par l'isoforme Kv1.5.

Ils sont présentés comme utiles dans une large gamme d'indications qui n'incluent pas le traitement de la dépression et de la douleur.

Il est important de mentionner que les composés de la présente invention n'interagissent pas avec les canaux potassiques et en particulier avec ceux du type Kv1.5 De plus, l'activité antagoniste NMDA des composés de l'invention s'avère très sensible aux modifications structurales des composés de formule (1). Ainsi, l'activité antagoniste NMDA est abolie lorsque :

1) la fonction 1-carboxamide est réduite en une fonction 1-aminométhyle, telle que celle des composés cyclobutane du brevet WO 2003/063797 ;
2) le groupe amino en position 3 sur le cyclobutane est différent d'un groupe amine primaire ($NH_2$). Dans la demande de brevet WO 2003063797, le groupe 3-amino est substitué par un groupe C(G)=NCN ;
3) la stéréochimie "cis" entre les groupes 1-aryle et 3-amino n'est pas respectée. En effet, lorsque les groupes 1-aryle et 3-amino sont de stéréochimie "trans", les composés correspondants sont dépourvus d'affinité pour le récepteur NMDA.

L'état de la technique est aussi représenté par les dérivés décrits dans la demande de brevet WO 99/52848 et répondant à la formule suivants :

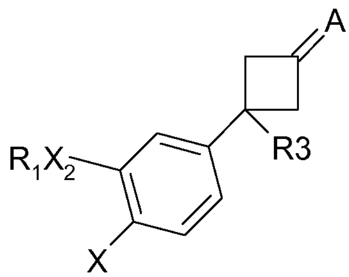

dans laquelle :

X est différent d'un atome d'hydrogène ;
A peut être un groupe $NR_7$ avec $R_7$ différent d'un atome d'hydrogène ;
R3 peut être un groupe $C(O)NR_8R_{10}$ dans lequel $R_8$ et $R_{10}$ peuvent être des chaînes alkyle en $C_1$-$C_4$. Lesdits composés sont revendiqués comme étant des inhibiteurs sélectifs des phosphodiestérases de type 4, utiles pour traiter les maladies inflammatoires et auto-immunes. Les composés de la présente invention se distinguent donc de ceux décrits dans la demande WO 99/52848 à la fois par leur structure chimique et leur activité pharmacologique.

L'état de la technique est également représenté par les dérivés décrits dans la demande de brevet WO

2010/112597 et ayant pour formule générale :

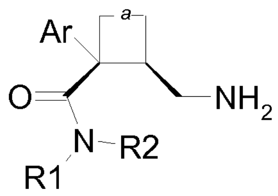

dans laquelle :

- a peut être une liaison simple ;
- Ar représente un groupe phényle substitué ou non, ou un noyau pyridine-3-yle substitué par un ou plusieurs atomes d'halogène ou groupes alkyle ou groupes alcoxyde ou par un groupe cyano ;
- R1 et R2 peuvent représenter ensemble ou indépendamment un groupe alkyle en $C_1$-$C_6$.

A la différence des composés de la demande de brevet WO 2010/112597, ceux de la présente invention n'ont pas d'affinité pour les sites de recapture de la sérotonine et de la noradrénaline. Les composés de formule (1) se différencient donc de ceux décrits dans la demande WO 2010/112597 non seulement au niveau de leur structure chimique mais aussi au niveau de leur activité pharmacologique.

L'état de la technique est enfin représenté par les composés décrits dans la demande de brevet WO 2000/051607 et ayant pour formule générale :

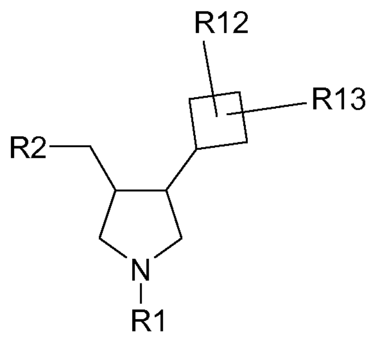

dans laquelle R12 et R13 représente un groupe alkyle en $C_{1-6}$, ou un groupe alcényle en $C_{2-6}$ ou un groupe alcynyle en $C_{2-6}$ substitué ou non.

Lesdits dérivés sont des modulateurs de chimiokines utiles dans la prévention ou le traitement de certaines maladies inflammatoires ou du système immunitaire. Là encore, les composés de la présente invention se distinguent donc de ceux décrits dans la demande WO 2000/051607, à la fois par leur structure chimique et leur activité pharmacologique.

La présente invention s'étend également aux sels des dérivés de formule générale (1) avec des acides organiques ou minéraux pharmaceutiquement acceptables. Dans la présente invention, le terme "pharmaceutiquement acceptable" se réfère à des entités moléculaires et des compositions qui ne produisent aucun effet adverse, allergique ou autre réaction indésirable quand elles sont administrées à un humain. Quand utilisé ici, le terme "excipient pharmaceutiquement acceptable" inclut tout diluant, adjuvant ou excipient, tel que des agents préservatifs, des agents de remplissage, des agents désintégrant, mouillant, émulsifiant, dispersant, antibactérien ou antifongique, ou bien encore des agents qui permettraient de retarder l'absorption et la résorption intestinale et digestive. L'utilisation de ces milieux ou vecteurs est bien connue de l'homme du métier. On désigne par "sels pharmaceutiquement acceptables" d'un composé les sels définis ici et qui possèdent l'activité pharmacologique du composé parent. De tels sels comprennent : les sels d'addition d'acide formés avec des acides minéraux, tels que l'acide chlorhydrique, l'acide bromhydrique, l'acide sulfurique, l'acide nitrique, l'acide phosphorique et similaires, ou formés avec des acides organiques, tels que l'acide acétique, l'acide benzènesulfonique, l'acide benzoïque, l'acide camphresulfonique, l'acide citrique, l'acide éthanesulfonique, l'acide fumarique, l'acide glucoheptonique, l'acide gluconique, l'acide glutamique, l'acide glycolique, l'acide hydroxynaphtoïque, l'acide 2-hydroxyéthanesulfonique, l'acide lactique, l'acide maléique, l'acide malique, l'acide mandélique, l'acide méthanesulfonique, l'acide muconique, l'acide 2-naphtalènesulfonique, l'acide propionique, l'acide salicylique, l'acide succinique, l'acide dibenzoyl-L-tartrique, l'acide tartrique, l'acide p-toluènesulfonique, l'acide triméthylacétique, l'acide trifluoroacétique et similaires.

Les sels pharmaceutiquement acceptables comprennent également les formes d'addition de solvants (solvates) ou les formes cristallines (polymorphes), telles que définies ici, du même sel d'addition d'acide.

La présente invention concerne également les composés de formule (1) ainsi que leurs sels pharmaceutiquement acceptables pour leur utilisation en tant que médicament.

La présente invention concerne les composés de formule (1) ainsi que leurs sels pharmaceutiquement acceptables pour leur utilisation en tant qu'antagonistes des récepteurs NMDA.

La présente invention concerne également les composés de formule (1) ainsi que leurs sels pharmaceutiquement acceptables pour leur utilisation en tant que médicaments destinés au traitement et/ou à la prévention de la dépression.

La présente invention concerne aussi les composés de formule (1) ainsi que leurs sels pharmaceutiquement acceptables pour leur utilisation en tant que médicaments destinés au traitement de la douleur, notamment les douleurs par excès de nociception, les douleurs neuropathiques et les douleurs mixtes.

Parmi les douleurs potentiellement sensibles à l'action des composés de formule générale (1), on peut citer plus particulièrement, à titre d'exemples illustratifs mais non limitatifs :

- les douleurs des neuropathies périphériques ou centrales résultant de lésions nerveuses d'origine traumatique (par exemple accident vasculaire cérébral), métabolique (par exemple, diabète), infectieuse (par exemple, VIH, zona, herpès), les névralgies du trijumeau, les douleurs dues aux chimiothérapies et/ou aux radiothérapies ;
- les douleurs inflammatoires, par exemple l'arthrite rhumatoïde, l'arthrite septique, l'ostéoarthrite, la polyarthrite, la goutte, les spondylarthrites, les rhumatismes abarticulaires aigus, les douleurs viscérales, par exemple le syndrome du côlon irritable, la maladie de Crohn ;
- les douleurs par excès de nociception, telles que les douleurs post-traumatiques, postopératoires, les brûlures, les torsions/distensions, les crises de colique néphrétique ou hépatiques, les douleurs articulaires, l'arthrose, les spondylarthropathies ;
- les douleurs mixtes, telles que les douleurs cancéreuses, les douleurs du dos et du bas du dos, ou d'autres douleurs difficiles à classer comme les céphalées, la fibromyalgie, les douleurs associées à des problèmes vasculaires/ischémiques comme l'angine de poitrine, la maladie de Reynaud.

La présente invention concerne également les composés de formule (1) ainsi que leurs sels pharmaceutiquement acceptables pour leur utilisation en tant que médicament destiné au traitement et/ou à la prévention de l'inflammation au niveau des articulations. Parmi les inflammations potentiellement sensibles à l'action des composés de formule générale (1), on peut citer plus particulièrement, à titre d'exemples illustratifs mais non limitatifs . les monoarthrites inflammatoires, l'arthrite rhumatoïde, l'arthrite septique, l'ostéoarthrite, la polyarthrite rhumatoïde, la goutte, la spondylarthrite, les rhumatismes abarticulaires aigus.

La présente invention concerne en outre une composition pharmaceutique caractérisée en ce qu'elle contient à titre de principe actif au moins un composé de formule générale (1) ou un de ses sels pharmaceutiquement acceptables.

L'invention s'étend également à une composition pharmaceutique caractérisée en ce qu'elle comprend au moins un composé de formule générale (1) ou l'un de ses sels pharmaceutiquement acceptables et au moins un excipient pharmaceutiquement acceptable.

L'invention s'étend également à une composition pharmaceutique pour son utilisation en tant que médicament destiné au traitement et/ou à la prévention de la dépression.

L'invention s'étend aussi à une composition pharmaceutique pour son utilisation en tant que médicament destiné au traitement de la douleur, notamment les douleurs par excès de nociception, les douleurs neuropathiques et mixtes.

Les compositions pharmaceutiques selon la présente invention peuvent être formulées pour l'administration à l'homme. Ces compositions sont réalisées de façon à pouvoir être administrées par voie orale, sublinguale, sous-cutanée, intramusculaire, intraveineuse, transdermique, locale ou rectale. Dans ce cas, l'ingrédient actif peut être administré sous formes unitaires d'administration, en mélange avec des supports pharmaceutiques classiques, aux êtres humains. Les formes unitaires d'administration appropriées comprennent les formes par voie orale, telles que les comprimés, les gélules, les poudres, les granules et les solutions ou suspensions orales, les formes d'administration sublinguale et buccale, les formes d'administration sous-cutanée, topique, intramusculaire, intraveineuse, intra-nasale ou intraoculaires et les formes d'administration rectale.

Avantageusement, la composition pharmaceutique selon la présente invention est formulée pour une administration par voie orale ou topique. Une administration par voie topique est la voie préférée pour le traitement de certains types de douleurs, telles que par exemple les douleurs articulaires.

On entend par administration topique une administration locale sur la peau ou une muqueuse.

Les formulations appropriées pour la forme d'administration choisie sont connues par l'homme du métier et

décrites, par exemple dans : Remington, The science and Practice of Pharmacy, 19e édition, 1995, Mack Publishing Company.

Lorsque l'on prépare une composition solide sous forme de comprimés, on mélange l'ingrédient actif principal avec un véhicule pharmaceutique, tel que la gélatine, l'amidon, le lactose, le stéarate de magnésium, le talc, la gomme arabique, la silice ou analogue. On peut enrober les comprimés de saccharose ou d'autres matières appropriées, ou encore on peut les traiter de telle sorte qu'ils aient une activité prolongée ou retardée et qu'ils libèrent d'une façon continue une quantité prédéterminée de principe actif.

On obtient une préparation en gélules en mélangeant l'ingrédient actif avec un diluant et en versant le mélange obtenu dans des gélules molles ou dures.

Une préparation sous forme de sirop ou d'élixir peut contenir l'ingrédient actif conjointement avec un édulcorant, un antiseptique, ainsi qu'un agent donnant du goût et un colorant approprié.

Les poudres ou les granules dispersibles dans l'eau peuvent contenir l'ingrédient actif en mélange avec des agents de dispersion ou des agents mouillants, ou des agents de mise en suspension, de même qu'avec des correcteurs du goût ou des édulcorants.

Pour une administration rectale, on recourt à des suppositoires qui sont préparés avec des liants fondant à la température rectale, par exemple du beurre de cacao ou des polyéthylèneglycols.

Pour une administration parentérale (intraveineuse, intramusculaire, intradermique, sous-cutanée), intra-nasale ou intraoculaire, on utilise des suspensions aqueuses, des solutions salines isotoniques ou des solutions stériles et injectables qui contiennent des agents de dispersion et/ou des agents mouillants pharmacologiquement compatibles.

Le principe actif peut également être formulé sous forme de microcapsules, éventuellement avec un ou plusieurs supports additifs.

L'administration topique de la composition pharmaceutique peut être accomplie par application d'une solution, d'une suspension, d'un gel, d'une lotion, d'un lait, d'une pommade, d'un onguent, d'une crème, d'un collyre, ou un autre véhicule utilisé pour une application topique bien connu de l'homme du métier. L'un des moyens possibles est l'administration de la composition pharmaceutique par un spray aérosol permettant de vaporiser un liquide en fines gouttelettes pour une distribution sur toute la surface à traiter ou, au contraire, de limiter avec précision une zone particulière à traiter ou encore sous forme solide, sous forme de stick. Un autre exemple de réalisation est le patch ou timbre qui fournit une libération continue de la composition topique. Le patch peut avoir un réservoir et une membrane poreuse ou une matrice solide qui sont bien connus de l'homme de l'art. D'autres modes d'administration, tels que l'iontophorèse ou l'électroporation, sont aussi utilisables.

Les compositions décrites dans la présente invention peuvent comprendre en plus des ingrédients ou composants habituellement mélangés dans de telles préparations topiques, par exemple les compositions peuvent aussi inclure des ingrédients additionnels, tels que des transporteurs, des hydratants, des huiles, des graisses, des cires, des surfactants, des agents épaississants, des antioxydants, des stabilisateurs de viscosité, des agents chélateurs, des tampons, des préservateurs, des parfums, des colorants, des humectants, des émollients, des dispersants, des crèmes solaires avec des composés bloquant les radiations et particulièrement des bloqueurs d'UV, des antibactériens, des antifongiques, des désinfectants, des vitamines, des antibiotiques ou d'autres agents anti-acné, ainsi que d'autres substances adaptées qui n'ont pas d'effets adverses néfastes sur l'activité de la composition topique. Par exemple des ingrédients additionnels peuvent être utilisés, comme le phosphate acide de sodium, l'extrait d'Hamamélis, la glycérine, l'huile de noyaux d'abricot, l'huile de maïs. En plus des composants décrits ci-dessus, les compositions de la présente invention peuvent contenir optionnellement d'autres ingrédients. Par exemple, la triéthanolamine peut être ajoutée comme agent de réticulation. Un préservateur comme l'hydroxytoluène butylé peut aussi être ajouté. D'autres agents réduisant l'irritation peuvent aussi être ajoutés ; à cet égard, on peut mentionner sans être limitatif le glycérol. Pour une administration topique, les compositions peuvent contenir des émollients et émulsifiants conventionnels incluant des alginates, le stéarate de glycéryle, le PEG-100 stéarate, l'alcool cétylique, le propylparabène, le butylparabène les sorbitols, le monostéarate d'anhydrosorbitol éthoxylé (TWEEN), le pétrolatum blanc (VASELINE), la triéthanolamine, l'huile d'émeu, des extraits d'aloe vera, la lanoline, le beurre de cacao et d'autres.

Les compositions décrites peuvent être appliquées sur la surface à traiter de la peau du patient. La fréquence d'application dépendra des circonstances et du patient. Par exemple, les compositions peuvent être appliquées quotidiennement, deux fois par jour ou même plus fréquemment.

Les dosages d'un composé de formule générale (1) ou de l'un de ses sels pharmaceutiquement acceptables dans les compositions de l'invention peuvent être ajustés afin d'obtenir une quantité de substance qui est efficace pour obtenir la réponse thérapeutique désirée pour une composition particulière à la méthode d'administration. La dose efficace du composé de l'invention varie en fonction de nombreux paramètres, tels que, par exemple, la voie d'administration choisie, le poids, l'âge, le sexe, la nature de la pathologie, la sensibilité de l'individu à traiter. En conséquence, la posologie optimale devra être déterminée par le spécialiste en la matière en fonction des paramètres qu'il juge pertinents. Bien que les doses efficaces puissent varier dans de larges proportions, les doses journalières pourraient s'échelonner entre 1 mg et 1000 mg par 24 h pour un adulte d'un poids moyen de 70 kg, en une ou plusieurs prises.

L'invention inclut enfin la méthode de synthèse de produits de composés de formule générale (1) ainsi que celles des intermédiaires de synthèses de formules (C) et (D).

Les composés de formule générale (1) peuvent être obtenus par le procédé décrit dans le schéma réactionnel illustré ci-après.

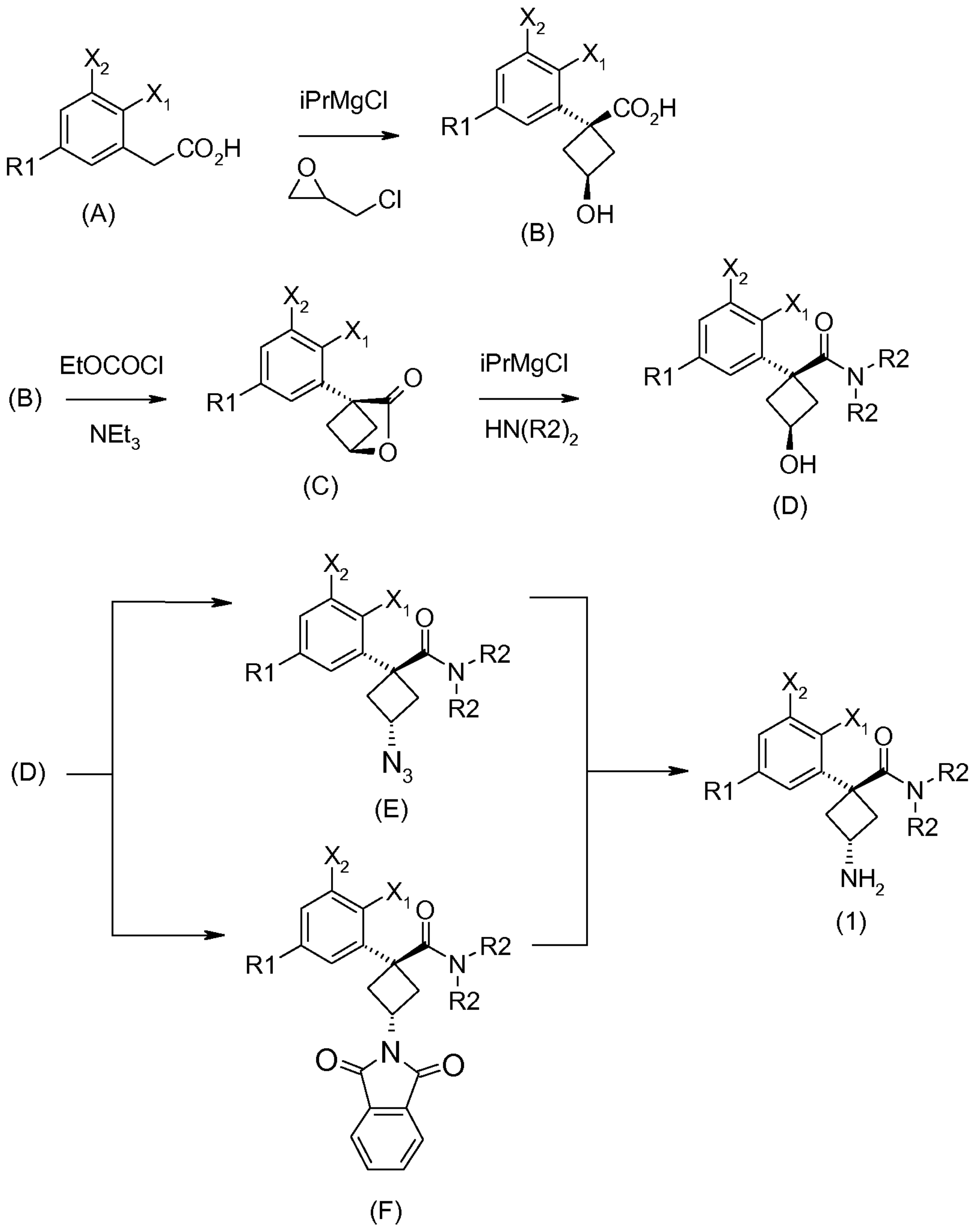


(A) (B) (C) (D) (E) (F) (1)

Schéma réactionnel

La préparation des composés de l'invention utilise comme matière première les dérivés de l'acide benzèneacétique, de formule (A), disponibles commercialement comme : l'acide benzèneacétique (RN 103-82-2) ; l'acide 2-fluorobenzèneacétique (RN 451-82-1) ; l'acide 3-fluorobenzèneacétique (RN 331-25-9) ; l'acide 3-chlorobenzèneacétique (RN 1878-65-5) ; l'acide 3-méthylbenzèneacétique (RN 621-36-3) ; l'acide 3-cyanobenzèneacétique (RN 1878-71-3) ; l'acide 3-méthoxybenzèneacétique (RN 1798-09-0) ; l'acide 2,5-difluorobenzèneacétique (RN 85068-27-5) ; l'acide 3,5-difluorobenzèneacétique (RN 105184-38-1) ; l'acide 3,5-dichlorobenzèneacétique (RN 51719-65-4) ; l'acide 2-fluoro-3-chlorobenzèneacétique (RN 261762-96-3). Les dérivés de formule (A) sont condensés avec l'épichlorhydrine selon une méthode adaptée de celle décrite dans la demande de brevet WO 2007/038452 pour donner les dérivés de formule (B)

dans lesquels les fonctions alcool et acide carboxylique sont de stéréochimie 'cis'. Ledit brevet ne décrit pas les intermédiaires de formule (B). Les lactones de formule (C) sont ensuite formées à partir des dérivés de formule (B) en utilisant une méthode classique d'activation de la fonction acide, telle que, par exemple, au moyen d'un chloroformiate d'alkyle, tel que décrit dans la demande WO 2008/092955. L'ouverture de la lactone de formule (C) est ensuite avantageusement réalisée au moyen du sel de magnésium de l'amine secondaire appropriée selon Williams et al. (Tetrahedron Lett., 1995, 36, 5461-5464) pour conduire au carboxamide correspondant de formule (D). L'introduction de la fonction amine primaire en position 3 du cyclobutane avec inversion de la stéréochimie peut être effectuée par l'intermédiaire de l'azide de formule (E) selon Soltani Rad et al. (Tetrahedron Lett., 2007, 48, 3445-3449). La réduction de la fonction azido en l'amine primaire correspondante est alors accomplie soit par hydrogénation catalytique, soit par une réaction de Staudinger. Alternativement, la conversion du composé de formule (D) en l'amine de formule (1) peut être réalisée par l'intermédiaire du phtalimide de formule (F) selon la méthode classique de Gabriel (par exemple, demande WO 2006081179).

Les exemples suivants illustrent l'invention mais ne la limitent en aucune façon. Dans les exemples ci-après :

(i) des formes cristallines différentes peuvent donner des points de fusion différents ; les points de fusion rapportés dans la présente demande sont ceux des produits préparés selon la méthode décrite et ne sont pas corrigés ;
(ii) la structure des produits obtenus selon l'invention est confirmée par les spectres de résonance magnétique nucléaire (RMN), et spectrométrie de masse ; la pureté des produits finaux est vérifiée par CCM et l'analyse centésimale ;
(iii) les spectres RMN sont enregistrés dans le solvant indiqué ; les déplacements chimiques ($\delta$) sont exprimés en partie par million (ppm) par rapport au tétraméthylsilane ; la multiplicité des signaux est indiquée par : s, singulet ; d, doublet ; t, triplet ; q, quadruplet ; qu, quintuplet, m, multiplet ; 1, large ;
(iv) les différents symboles des unités ont leur signification habituelle : $\mu$g (microgramme) ; mg (milligramme) ; g (gramme) ; mL (millilitre) ; mV (millivolt) ; °C (degré Celsius) ; mmole (millimole) ; nmole (nanomole) ; cm (centimètre) ; nm (nanomètre) ; min (minute) ; ms (milliseconde), Hz (hertz) ;
(v) les abréviations ont la signification suivante : F (point de fusion) ; Eb (point d'ébullition) ;
(vi) par "température ambiante", on entend une température comprise entre 20°C et 25°C.

Exemple 1 : *trans*-3-amino-*N,N*-diéthyl-1-phénylcyclobutanecarboxamide (1a1)

Etape 1 : acide cis-1-phényl-3-hydroxy-cyclobutanecarboxylique (B1)

Dans un tricol, introduire 2,2 eq de chlorure d'isopropylmagnésium et refroidir le milieu réactionnel à 0°C. Ajouter 1 eq d'acide phénylacétique dilué dans du THF, la température doit être maintenue entre 40 et 50°C. Refroidir le milieu à 20°C et ajouter 1,8 eq d'épichlorhydrine ; la température doit être maintenue entre 20 et 25°C, et laisser sous agitation à cette température pendant 45 min. Ajouter ensuite 2 eq de chlorure d'isopropylmagnésium (2M dans le THF) goutte à goutte et laisser sous agitation à température ambiante pendant 2 h. Chauffer ensuite le milieu réactionnel à 60°C pendant 19 h. Laisser refroidir le milieu et l'acidifier avec une solution d'HCl (1N) jusqu'à pH 1. Ajouter du dichlorométhane (DCM) et extraire. Décanter, sécher la phase organique sur du $MgSO_4$, puis évaporer le DCM sous pression réduite. Purifier le résidu par flash-chromatographie avec comme éluant : DCM, puis DCM/méthanol 70:30. Le produit du titre est obtenu sous la forme d'un solide jaune pâle (rendement = 70 %). $C_{11}H_{12}O_3$ (poids moléculaire = 192).
$^1$H-RMN (DMSO $d_6$, 400 MHz) $\delta$ (ppm) : 2,50 (m, 2H), 2,74 (t, 2H, J = 9, 4 Hz), 3, 32 (s, 1H), 3, 85 (qu, 1H, J = 7, 2 Hz), 7,22-7,38 (m, 5H), 12,21 (s, 1H).
SM-ESI : 193, 1 ($MH^+$).

Etape 2 : 4-phényl-2-oxabicyclo[2.1.1]hexane-3-one (C1)

Dans un ballon, introduire 1 eq du composé (B1), dilué dans du THF, et 1,03 eq de triéthylamine. Laisser sous agitation à température ambiante jusqu'à dissolution, puis refroidir le milieu réactionnel à 0°C. Ajouter 1,03 eq de chloroformiate d'éthyle et agiter à cette température pendant 1 h, puis revenir à température ambiante et laisser sous agitation pendant 20 h. Evaporer le THF sous pression réduite, reprendre le résidu obtenu par de l'eau et extraire avec de l'acétate d'éthyle (AcOEt). Décanter, sécher l'acétate sur $MgSO_4$ puis l'évaporer sous pression réduite. Purifier le résidu par flash-chromatographie avec comme éluant : heptane, puis heptane/AcOEt 60:40. Le produit du titre est obtenu sous la forme d'une huile incolore (rendement = 87 %).
$C_{11}H_{10}O_2$ (PM = 174).
$^1$H-RMN ($CDCl_3$, 400 MHz) $\delta$ (ppm) : 2,71 (m, 2H), 2,89 (m, 2H), 4,97 (s, 1H), 7,31-7,42 (m, 5H).
SM-ESI : 175 ($MH^+$).

Etape 3 : *cis*-3-hydroxy-*N,N*-diéthyl-1-phénylcyclobutanecarboxamide (D1a)

Dans un tricol sous atmosphère d'azote, introduire 1 eq du composé (C1), 2 eq de diéthylamine et le THF. Refroidir le milieu réactionnel à -20°C, puis couler goutte à goutte les 3 eq de chlorure d'isopropylmagnésium (2M dans le THF) en maintenant la température inférieure à -5°C. Laisser le mélange sous agitation pendant 2 h à une température comprise entre -10 et -20°C. Hydrolyser le milieu réactionnel avec une solution de NaCl saturée, puis ajouter une solution d'HCl (1N) et extraire avec de l'AcOEt. Sécher la phase organique sur $MgSO_4$, filtrer et concentrer. Purifier le résidu par flash-chromatographie avec comme éluant un mélange : DCM/méthanol 85:15. Le produit du titre est obtenu sous la forme d'un solide jaune pâle (rendement = 99 %).
$C_{15}H_{21}NO_2$ (PM = 247).
$^1$H-RMN ($CDCl_3$, 400 MHz) δ (ppm) : 0,63 (t, 3H, J = 7,2 Hz), 1,08 (t, 3H, J = 7,2 Hz), 2,72 (m, 2H), 2,82 (m, 2H), 2,90 (q, 2H, J = 7,2 Hz), 3,21 (q, 2H, J = 7,2 Hz), 4,36 (qu, 1H, J = 7,4 Hz), 7,21-7,36 (m, 5H). Le signal correspondant à l'H du OH n'est pas visible sur le spectre.
SM-ESI : 248 ($MH^+$).

Etape 4 : *trans*-3-azido-*N,N*-diéthyl-1-phénylcyclobutanecarboxamide (E1a)

Dans un ballon, introduire 1 eq du composé (D1a), 1,5 eq de N-(p-toluènesulfonyl)imidazole, 2 eq de triéthylamine, 0,025 eq d'iodure de tétrabutylammonium, 3 eq d'azide de sodium et le DMF. Agiter et chauffer le milieu réactionnel à 160°C pendant 4 h. Verser le milieu réactionnel sur de l'eau glacée et extraire avec de l'éther éthylique. Sécher la phase organique sur $MgSO_4$, filtrer et concentrer. Purifier le résidu par flash-chromatographie avec comme éluant un mélange : heptane/AcOEt 70:30. Le produit du titre est obtenu sous la forme d'une huile incolore (rendement = 65 %).
$C_{15}H_{20}N_4O$ (PM = 272).
$^1$H-RMN ($CDCl_3$, 400 MHz) δ (ppm) : 0,52 (t, 3H, J = 7,2 Hz), 1,11 (t, 3H, J = 7,2 Hz), 2,47 (m, 2H), 2,89 (q, 2H, J = 7,2 Hz), 3,14 (m, 2H), 3,34 (q, 2H, J = 7,2 Hz), 3,96 (qu, 1H, J = 7,8 Hz), 7,23 (m, 3H), 7,35 (m, 2H).
SM-ESI: 273 ($M+H^+$).

Etape 5 : *trans*-3-amino-*N,N*-diéthyl-1-phénylcyclobutanecarboxamide (1a1)

Dans un ballon, dissoudre 1 eq du composé (E1a) dans du méthanol. Dégazer la solution pendant 30 min avec de l'azote, puis ajouter le Pd/C (20 % massique). Purger le système (cycle vide/$H_2$ gaz) et hydrogéner le milieu réactionnel pendant 3 h à température ambiante sous agitation. Filtrer le catalyseur et évaporer le solvant. Purifier le résidu obtenu par flash-chromatographie avec comme éluant un mélange : DCM/méthanol/$NH_4OH$: 90:9:1. Le produit du titre est obtenu sous la forme d'une huile incolore (rendement = 70 %).
$C_{15}H_{22}N_2O$ (PM = 246).
$^1$H-RMN (DMSO $d_6$, 400 MHz) δ (ppm) : 0,50 (t, 3H, J = 7,2 Hz), 1,10 (t, 3H, J = 7,2 Hz), 2,11 (m, 2H), 2,92 (q, 2H, J = 6,8 Hz), 3,12 (m, 2H), 3,32 (q, 2H, J = 6,8 Hz), 3,46 (qu, 1H, J = 8,0 Hz), 7,18-7,35 (m, 5H). Le signal correspondant aux H du $NH_2$ n'est pas visible sur le spectre. SM-ESI : 247 ($MH^+$).
Maléate du composé du titre

La salification du composé précédent au moyen d'acide maléique permet d'obtenir le maléate du composé du titre sous la forme d'une poudre blanche.
F : 185°C.
$^1$H-RMN (DMSO $d_6$, 400 MHz) δ (ppm) : 0,42 (t, 3H, J = 7,0 Hz), 1,02 (t, 3H, J = 7,0 Hz), 2,56 (m, 2H), 2,85-2,96 (m, 4H), 3,25 (q, 2H, J = 6,8 Hz), 3,54 (qu, 1H, J = 8,4 Hz), 6,03 (s, 2H), 7,26 (m, 3H), 7,39 (t, 2H, J = 7,6 Hz), 8,00 (s, 2H). Le signal correspondant aux H du $NH_2$ n'est pas visible sur le spectre.
$^{13}$C-RMN (DMSO $d_6$, 100 MHz) δ (ppm) : 12,02, 12,15, 36,93, 39, 19, 40, 07, 41, 19, 46, 61, 124, 87, 126, 51, 128, 71, 136, 02, 142,69, 167,19, 171,10.
% Théorique : C 62,97, H 7,23, N 7,73.
% Trouvé : C 63,00, H 7,17, N 7,78.

Exemple 2 : *trans*-3-amino-*N,N*-diméthyl-1-phénylcyclobutanecarboxamide (1a2)

Etape 3 : *cis*-3-hydroxy-*N,N*-diméthyl-1-phénylcyclobutanecarboxamide (D1b)

Identique à l'étape 3 décrite dans l'exemple 1, en utilisant la diméthylamine au lieu de la diéthylamine. Le produit du titre est obtenu sous la forme d'une huile incolore (rendement = 89 %).
$^1$H-RMN ($CDCl_3$, 400 MHz) δ (ppm) : 2,65 (m, 2H), 2,55 (s, 3H), 2, 95 (s, 3H), 2,80 (m, 2H), 4,27 (qu, 1H, J = 7,8 Hz), 7,19-7,35 (m, 5H). Le signal correspondant à l'H du OH n'est pas visible sur le spectre.

Etape 4 : *trans*-3-azido-*N,N*-diméthyl-1-phénylcyclobutanecarboxamide (E1b)

Identique à l'étape 4 de l'exemple 1. Le produit du titre est obtenu sous forme d'un solide beige (rendement = 95 %).
$^1$H-RMN ($CDCl_3$, 400 MHz) $\delta$ (ppm) : 2,50 (m, 2H), 2,54 (s, 3H), 2, 96 (s, 3H), 3,18 (m, 2H), 3, 97 (qu, 1H, J = 7,8 Hz), 7,24 (m, 3H), 7,36 (m, 2H).

Etape 5 : *trans*-3-amino-*N,N*-diméthyl-1-phénylcyclobutanecarboxamide (1a2)

Identique à l'étape 5 décrite dans l'exemple 1. Le produit du titre est obtenu sous la forme d'une huile incolore (rendement = 84 %).
$C_{13}H_{18}N_2O$ (PM = 218).
$^1$H-RMN ($CDCl_3$, 400 MHz) $\delta$ (ppm) : 2,13 (m, 2H), 2,55 (s, 3H), 2, 95 (s, 3H), 3, 15 (m, 2H), 3, 47 (qu, 1H, J = 7,8 Hz), 7,19-7,35 (m, 5H). Le signal correspondant aux H du $NH_2$ n'est pas visible sur le spectre.
SM-ESI : 219 ($MH^+$).
Maléate du composé du titre
La salification du composé précédent au moyen d'acide maléique permet d'obtenir le maléate du composé du titre sous la forme d'une poudre blanche.
F : 163°C.
$^1$H-RMN (DMSO $d_6$, 400 MHz) $\delta$ (ppm) : 2,51 (m, 5H), 2,86 (s, 3H), 2, 98 (m, 2H), 3, 36 (s, 1H), 3, 53 (qu, 1H, J = 8,4 Hz), 6,03 (s, 2H), 7,26 (m, 3H), 7,39 (t, 2H, J = 7,6 Hz), 8,05 (s, 3H).
$^{13}$C-RMN (DMSO $d_6$, 100 MHz) $\delta$ (ppm) : 35,80, 37,20, 37,34, 39,91, 46,54, 124,94, 126,59, 128,72, 136,00, 142,43, 167,15, 171,68.
% Théorique : C 61,07, H 6,63, N 8,38.
% Trouvé . C 60,73, H 6,43, N 8,15.

Exemple 3 : *trans*-3-amino-*N,N*-diéthyl-1-(2-fluorophényl)-cyclobutanecarboxamide (1b)

Etape 1 : acide *cis*-3-hydroxy-1-(2-fluorophényl)-cyclobutanecarboxylique (B2)

Identique à l'étape 1 décrite dans l'exemple 1, en utilisant l'acide 2-fluorophénylacétique comme produit de départ. Le produit du titre est obtenu sous la forme d'un solide blanc (rendement = 49 %).
$C_{11}H_{11}FO_3$ (PM = 210).
$^1$H-RMN ($CDCl_3$, 400 MHz) $\delta$ (ppm) : 2,80 (m, 2H), 2,97 (m, 2H), 4,29 (qu, 1H, J = 6,4 Hz), 7, 04-7, 23 (m, 4H). Les signaux correspondant aux H des OH de l'alcool et de l'acide ne sont pas visibles sur le spectre.
SM-ESI : 211 ($MH^+$).

Etape 2 : 4-(2-fluorophényl)-2-oxabicyclo[2.1.1]hexane-3-one (C2)

Identique à l'étape 2 décrite dans l'exemple 1. Le produit du titre est obtenu sous la forme d'une huile incolore (rendement = 81 %).
$C_{11}H_9FO_2$ (PM = 192).
$^1$H-RMN ($CDCl_3$, 400 MHz) $\delta$ (ppm) : 2,75 (m, 2H), 2,99 (m, 2H), 5,01 (s, 1H), 7,07-7,42 (m, 4H).
SM-ESI : = 193 ($MH^+$).

Etape 3 : *cis*-3-hydroxy-*N,N*-diéthyl-1-(2-fluorophényl)-cyclobutanecarboxamide (D2a)

Identique à l'étape 3 de l'exemple 1. Le produit du titre est obtenu sous la forme d'un solide blanc (rendement = 85 %).
$C_{15}H_{20}NO_2F$ (PM = 265).
$^1$H-RMN ($CDCl_3$, 400 MHz) $\delta$ (ppm) : 0,47 (t, 3H, J = 6,8 Hz), 1,10 (t, 3H, J = 6,8 Hz), 2,77-2,89 (m, 4H), 2,95 (m, 2H), 3,31 (m, 2H), 4,32 (qu, 1H, J = 6,8 Hz), 7,04 (t, 1H, J = 7,8 Hz), 7,15 (t, 1H, J = 7,8 Hz), 7,26 (m, 1H), 7,37 (t, 1H, J = 7,8 Hz). Le signal correspondant à l'H du OH n'est pas visible sur le spectre.
SM-ESI : 266 ($MH^+$).

Etape 4 : *trans*-3-azido-*N,N*-diéthyl-1-(2-fluorophényl)-cyclobutanecarboxamide (E2a)

Identique à l'étape 4 décrite dans l'exemple 1. Le produit du titre est obtenu sous la forme d'une huile incolore

(rendement = 75 %).
$C_{15}H_{19}N_4OF$ (PM = 290).
$^1H$-RMN ($CDCl_3$ 400 MHz) δ (ppm) : 0,42 (t, 3H, J = 7,0 Hz), 1,10 (t, 3H, J = 7,0 Hz), 2,55 (m, 2H), 2,98 (q, 2H, J = 7,0 Hz), 3, 19 (m, 2H), 3,31 (q, 2H, J = 7,0 Hz), 4,02 (qu, 1H, J = 8,0 Hz), 7,03 (m, 1H), 7,14-7,29 (m, 3H).
SM-ESI : 291 ($MH^+$).

Etape 5 : *trans*-3-amino-*N,N*-diéthyl-1-(2-fluorophényl)-cyclobutanecarboxamide (1b)

Identique à l'étape 5 décrite dans l'exemple 1. Le produit du titre est obtenu sous la forme d'une huile incolore (rendement = 90 %).
$C_{15}H_{21}N_2OF$ (PM = 264).
$^1H$-RMN ($CDCl_3$, 400 MHz) δ (ppm) : 0,42 (t, 3H, J = 6,8 Hz), 1,10 (t, 3H, J = 6,8 Hz), 2,19 (m, 2H), 3,00 (q, 2H, J = 6,8 Hz), 3, 17 (m, 2H), 3,31 (q, 2H, J = 6,8 Hz), 3,53 (qu, 1H, J = 8,0 Hz), 7,00 (m, 1H), 7,11-7,31 (m, 3H). Les signaux correspondant aux H du $NH_2$ ne sont pas visibles sur le spectre.
SM-ESI : 265 ($MH^+$).

Maléate du composé du titre

La salification du composé précédent au moyen d'acide maléique permet d'obtenir le maléate du composé du titre sous la forme d'une poudre blanche.
F : 193°C.
$^1H$-RMN (DMSO $d_6$, 400 MHz) δ (ppm) : 0,01 (t, 3H, J = 6,8 Hz), 0,77 (t, 3H, J = 6,8 Hz), 2,36 (m, 2H), 2,72 (m, 4H), 2,97 (q, 2H, J = 6,8 Hz), 3,22 (s, 1H), 3,38 (qu, 1H, J = 8,0 Hz), 5,81 (s, 2H), 6,94 (m, 1H), 7,04-7,14 (m, 2H), 7,33 (m, 1H), 7,75 (s, 3H).
$^{13}C$-RMN (DMSO $d_6$, 100 MHz) δ (ppm) : 12,00, 12,20, 36,14, 39, 97, 40, 60, 41, 19, 43, 60, 115, 71 (d, $^2J_{C-F}$ = 21 Hz), 124,64 (d, $^4J_{C-F}$ = 4 Hz), 128, 00 (d, $^3J_{C-F}$ = 5 Hz), 128, 80 (d, $^3J_{C-F}$ = 8 Hz), 130, 07 (d, 2 $J_{C-F}$ = 13 Hz), 136, 04, 158, 52, 160, 96, 167,14, 169,93.
% Théorique : C 59,99, H 6,62, N 7,36.
% Trouvé : C 60,15, H 6,48, N 7,20.

Exemple 4 : *trans*-3-amino-*N,N*-diéthyl-1-(3-fluorophényl)-cyclobutanecarboxamide (1c)

Etape 1 : acide *cis*-3-hydroxy-1-(3-fluorophényl)-cyclobutanecarboxylique (B3)

Identique à l'étape 1 de l'exemple 1 en utilisant l'acide 3-fluorophénylacétique comme acide de départ. Le produit du titre est obtenu sous la forme d'un solide blanc (rendement = 52 %).
$C_{11}H_{11}FO_3$ (PM = 210).
$^1H$-RMN (DMSO $d_6$, 400 MHz) δ (ppm) : 2,50 (m, 2H), 2,75 (m, 2H), 3,86 (qu, 1H, J = 7,2 Hz), 5,18 (s, 1H), 7,07-7,21 (m, 3H), 7,40 (m, 1H), 12,40 (s, 1H).
SM-ESI : 211 ($MH^+$).

Etape 2 . 4-(3-fluorophényl)-2-oxabicyclo[2.1.1]hexane-3-one (C3)

Identique à l'étape 2 décrite dans l'exemple 1. Le produit du titre est obtenu sous la forme d'une huile incolore (rendement = 91 %).
$C_{11}H_9O_2F$ (PM = 192).
$^1H$-RMN (DMSO $d_6$, 400 MHz) δ (ppm) : 2,83 (s, 4H), 5,09 (s, 1H), 7,15-7,22 (m, 3H), 7,38-7,47 (m, 1H).
SM-ESI : 193 ($MH^+$).

Etape 3 : *cis*-3-hydroxy-*N,N*-diéthyl-1-(3-fluorophényl)-cyclobutanecarboxamide (D3a)

Identique à l'étape 3 de l'exemple 1. Le produit du titre est obtenu sous la forme d'un solide blanc (rendement = 92 %).
$C_{15}H_{20}NO_2F$ (PM = 265).
$^1H$-RMN (DMSO $d_6$, 400 MHz) δ (ppm) : 0,62 (t, 3H, J = 7,2 Hz), 0,97 (t, 3H, J = 7,2 Hz), 2,50 (m, 2H), 2,66 (m, 2H), 2,86 (q, 2H, J = 7,2 Hz), 3,19 (q, 2H, J = 7,2 Hz), 4,05 (m, 1H), 5,12 (d, 1H, J = 6,8 Hz), 7,04-7,15 (m, 3H), 7,39 (m, 1H).
SM-ESI : 266 ($MH^+$).

Etape 4 : *trans*-3-azido-*N,N*-diéthyl-1-(3-fluorophényl)-cyclobutanecarboxamide (E3a)

Identique à l'étape 4 décrite dans l'exemple 1. Le produit du titre est obtenu sous la forme d'une huile incolore (rendement = 72 %).
$^{1}$H-RMN (DMSO $d_6$, 400 MHz) δ (ppm) : 0,59 (t, 3H, J = 7,2 Hz), 1,00 (t, 3H, J = 7,2 Hz), 2,40 (m, 2H), 2,86 (q, 2H, J = 7,2 Hz), 3,04 (m, 2H), 3,24 (q, 2H, J = 7,2 Hz), 4,07 (m, 1H), 7,04-7,15 (m, 3H), 7,39 (m, 1H).

Etape 5 : *trans*-3-amino-*N,N*-diéthyl-1-(3-fluorophényl)-cyclobutanecarboxamide (1c)

Identique à l'étape 5 décrite dans l'exemple 1. Le produit du titre est obtenu sous la forme d'une huile incolore (rendement = 93 %).
$C_{15}H_{21}N_2OF$ (PM = 264).
SM-ESI : 265 ($MH^+$).

Maléate du composé du titre

La salification du composé précédent au moyen d'acide maléique permet d'obtenir le maléate du composé du titre sous la forme d'une poudre blanche.
F : 174°C.
$^{1}$H-RMN (DMSO $d_6$, 400 MHz) δ (ppm) : 0,49 (t, 3H, J = 7,0 Hz), 1,02 (t, 3H, J = 7,0 Hz), 2,56 (m, 2H), 2,91 (m, 4H), 3,26 (q, 2H, J = 7,0 Hz), 3, 35 (s, 1H), 3,53 (qu, 1H, J = 8,4 Hz), 6,03 (s, 2H), 7,01 (d, 1H, J = 8,0 Hz), 7,09-7,20 (m, 2H), 7,42 (m, 1H), 7,99 (s, 3H).
$^{13}$C-RMN (DMSO $d_6$, 100 MHz) δ (ppm) : 12,10, 36, 93, 39, 23, 39, 91, 41,18, 46, 40, 111, 03 (d, $^{2}J_{C-F}$ = 22 Hz), 113,39 (d, $^{2}J_{C-F}$ = 21 Hz), 121, 11 (d, $^{4}J_{C-F}$ = 2 Hz), 130, 77 (d, $^{3}J_{C-F}$ = 9 Hz), 136, 02, 145, 55 (d, $^{3}J_{C-F}$ = 7 Hz), 161, 21, 163, 64, 167,14, 170,60.
% Théorique : C 59,99, H 6,62, N 7,36.
% Trouvé C 59,11, H 6,40, N 7,07.

Exemple 5 : *trans*-3-amino-*N,N*-diéthyl-1-(3-méthoxyphényl)-cyclobutanecarboxamide (1d)

Etape 1 : acide *cis*-3-hydroxy-1-(3-méthoxyphényl)-cyclobutanecarboxylique (B4)

Identique à l'étape 1 de l'exemple 1 en utilisant l'acide 3-méthoxyphénylacétique au lieu de l'acide phénylacétique. Le produit du titre est obtenu sous la forme d'un solide blanc (rendement = 50 %).
$C_{12}H_{14}O_4$ (PM = 222).
$^{1}$H-RMN (DMSO $d_6$, 400 MHz) δ (ppm) : 2,50 (m, 2H), 2,73 (m, 2H), 3,75 (s, 3H), 3,86 (qu, 1H, J = 7,2 Hz), 5,14 (s, 1H), 6, 82 (dd, 1H, J = 8,0 Hz et J = 2,0 Hz), 6, 87 (s, 1H), 6, 93 (d, 1H, J = 8,0 Hz), 7,26 (t, 1H, J = 8,0 Hz), 12,23 (s, 1H).
SM-ESI : 222.

Etape 2 : 4-(3-méthoxyphényl)-2-oxabicyclo[2.1.1]hexane-3-one (C4)

Identique à l'étape 2 décrite dans l'exemple 1. Le produit du titre est obtenu sous la forme d'une huile incolore (rendement = 85 %).
$C_{12}H_{12}O_2$ (PM = 188).
$^{1}$H-RMN (DMSO $d_6$, 400 MHz) δ (ppm) : 2,80 (m, 4H), 3,76 (s, 3H), 5,06 (s, 1H), 6,87-6,91 (m, 3H), 7,30 (t, 1H, J = 8,0 Hz).
SM-ESI : 189 ($MH^+$).

Etape 3 : *cis*-3-hydroxy-*N,N*-diéthyl-1-(3-méthoxyphényl)-cyclobutanecarboxamide (D4a)

Identique à l'étape 3 décrite pour l'exemple 1. Le produit du titre est obtenu sous la forme d'un solide blanc (rendement = 92 %).
$C_{16}H_{23}NO_3$ (PM = 277).
$^{1}$H RMN (DMSO $d_6$, 400 MHz) δ (ppm) : 0,62 (t, 3H, J = 6,8 Hz), 0,97 (t, 3H, J = 6,8 Hz), 2,50 (m, 2H), 2,64 (m, 2H), 2,86 (q, 2H, J = 6,8 Hz), 3,19 (q, 2H, J = 6,8 Hz), 3,73 (s, 3H), 4,06 (se, 1H, J = 7,6 Hz), 5,08 (d, 1H, J = 7,2 Hz), 6,81 (m, 2H), 6,89 (d, 1H, J = 7,6 Hz), 7,27 (m, 1H).
SM-ESI : 278 ($MH^+$).

Etape 4 : *trans*-3-azido-*N,N*-diéthyl-1-(3-méthoxyphényl)-cyclobutanecarboxamide (E4a)

Identique à l'étape 4 décrite pour l'exemple 1. Le produit du titre est obtenu sous la forme d'une huile incolore (rendement = 82 %).
$C_{16}H_{22}N_4O_2$ (PM = 302).
$^1$H-RMN (DMSO $d_6$, 400 MHz) δ (ppm) : 0,59 (t, 3H, J = 6,8 Hz), 1,00 (t, 3H, J = 6,8 Hz), 2,40 (m, 2H), 2,86 (q, 2H, J = 6,8 Hz), 3,05 (m, 2H), 3,24 (q, 2H, J = 6,8 Hz), 3,74 (s, 3H), 3, 95 (qu, 1H, J = 7,6 Hz), 6, 76 (m, 1H), 6, 83 (m, 2H), 7,30 (m, 1H).
SM-ESI : 303 ($MH^+$).

Etape 5 : *trans*-3-amino-*N,N*-diéthyl-1-(3-méthoxyphényl)-cyclobutanecarboxamide (1d)

Identique à l'étape 5 décrite pour l'exemple 1. Le produit du titre est obtenu sous la forme d'une huile incolore (rendement = 88 %).
$C_{16}H_{24}N_2O_2$ (PM = 276).
$^1$H-RMN (DMSO $d_6$, 400 MHz) δ (ppm) : 0,50 (t, 3H, J = 7,0 Hz), 1,00 (t, 3H, J = 7,0 Hz), 2,02 (m, 2H), 2,83 (m, 4H), 3,10 (qu, 1H, J = 8,0 Hz), 3,23 (q, 2H, J = 7,2 Hz), 3,33 (s, 2H), 3,73 (s, 3H), 6,73-6,79 (m, 3H), 7,25 (t, 1H, J = 8,0 Hz).
SM-ESI : 277 ($MH^+$).

Maléate du composé du titre

La salification du composé précédent au moyen d'acide maléique permet d'obtenir le maléate du composé du titre sous la forme d'une poudre blanche.
F : 156°C.
$^1$H-RMN (DMSO $d_6$, 400 MHz) δ (ppm) : 0,47 (t, 3H, J = 6,8 Hz), 1,02 (t, 3H, J = 6,8 Hz), 2,55 (m, 2H), 2,89 (m, 4H), 3,26 (q, 2H, J = 6,8 Hz), 3,35 (s, 1H), 3, 5 2 (qu, 1H, J = 8,4 Hz), 3,75 (s, 3H), 6,03 (s, 2H), 6,78-6,86 (m, 3H), 7,31 (t, 1H, J = 8,0 Hz), 7,98 (s, 3H).
$^{13}$C-RMN (DMSO $d_6$, 100 MHz) δ (ppm) : 12,11, 36,98, 39,23, 39,99, 41,23, 46,57, 55,03, 111,05, 111,54, 117,12, 129,89, 136,03, 144,22, 159,54, 167,12, 171,02.
% Théorique : C 61,21, H 7,19, N 7,14.
% Trouvé : C 61,38, H 7,09, N 6,98.

Exemple 6 : *trans*-3-amino-*N,N*-diéthyl-1-(3-chlorophényl)-cyclobutanecarboxamide (1e)

Etape 1 : acide *cis*-3-hydroxy-1-(3-chlorophényl)-cyclobutanecarboxylique (B5)

Identique à l'étape 1 décrite dans l'exemple 1 en utilisant l'acide 3-chlorophénylacétique comme acide de départ. Le composé du titre est obtenu sous la forme d'un solide blanc (rendement = 52 %).
$C_{11}H_{11}O_3Cl$ (PM = 226.5).
$^1$H-RMN (DMSO $d_6$, 400 MHz) δ (ppm) : 2,50 (m, 2H), 2,75 (m, 2H), 3,86 (qu, 1H, J = 7,2 Hz), 5,19 (s, 1H), 7,31-7,40 (m, 4H), 12,44 (s, 1H).

Etape 2 : 4-(3-chlorophényl)-2-oxabicyclo[2.1.1]hexane-3-one (C5)

Identique à l'étape 2 décrite dans l'exemple 1. Le produit du titre est obtenu sous la forme d'une huile incolore (rendement = 78 %).
$C_{11}H_9O_2Cl$ (PM = 208).
$^1$H-RMN (DMSO $d_6$, 400 MHz) δ (ppm) : 2,84 (m, 4H), 5,09 (s, 1H), 7,29-7,45 (m, 4H).
SM-ESI : 209 ($MH^+$).

Etape 3 : *cis*-3-hydroxy-*N,N*-diéthyl-1-(3-chlorophényl)-cyclobutanecarboxamide (D5a)

Identique à l'étape 3 décrite dans l'exemple 1. Le composé du titre est obtenu sous la forme d'un solide blanc (rendement = 99 %).
$C_{15}H_{20}NO_2Cl$ (PM = 281,5).
$^1$H-RMN (DMSO $d_6$, 400 MHz) δ (ppm) : 0,63 (t, 3H, J = 6,8 Hz), 0,96 (t, 3H, J = 6,8 Hz), 2,51 (m, 2H), 2,66 (m, 2H), 2,86 (q, 2H, J = 6,8 Hz), 3,19 (q, 2H, J = 6,8 Hz), 4,05 (qu, 1H, J = 7,6 Hz), 5,13 (s, 1H), 7,29-7,41 (m, 4H).
SM-ESI : 282,1 ($MH^+$).

Etape 4 : *trans*-3-(dioxoisoindoline-2-yl)-*N,N*-diéthyl-1-(3-chlorophényl)-cyclobutanecarboxamide (F5a).

Dans un ballon sous atmosphère d'azote, introduire 1 eq du composé (D5a), 1,1 eq de triphénylphosphine, 1,05 eq de phtalimide et le THF. Ajouter ensuite goutte à goutte 1,2 eq de diisopropyldiazodicarboxylate (DIAD) et laisser sous agitation à température ambiante pendant 16 h. Ajouter de l'eau et extraire avec du DCM. Sécher la phase organique sur $Na_2SO_4$, filtrer et concentrer. Purifier le résidu par flash-chromatographie avec comme éluant un mélange : heptane/AcOEt : 80:20. Le produit du titre est obtenu avec un rendement de 77 %.
$C_{23}H_{23}N_2O_3Cl$ (PM = 410,5).
$^1$H-RMN ($CDCl_3$, 400 MHz) $\delta$ (ppm) : 0,67 (t, 3H, J = 7,2 Hz), 1,18 (t, 3H, J = 7,2 Hz), 2,91 (q, 2H, J = 7,2 Hz), 3,11 (m, 2H), 3,35 (m, 2H), 3,42 (q, 2H, J = 7,2 Hz), 4,79 (qu, 1H, J = 8,8 Hz), 7,23 (m, 1H), 7,32 (m, 2H), 7,41 (s, 1H), 7,73 (m, 2H), 7,83 (m, 2H).
SM-ESI : 411,1 ($MH^+$).

Etape 5 : *trans*-3-amino-*N,N*-diéthyl-1-(3-chlorophényl)-cyclobutanecarboxamide (1e)

Introduire dans un ballon le dérivé (F5a) en solution dans l'éthanolamine. Chauffer le milieu réactionnel à 60°C pendant 1 h 30. Ajouter un mélange de glace et d'eau, agiter 15 min et extraire avec de l'AcOEt. Laver la phase organique avec une solution de NaCl saturée et décanter. Sécher la phase organique sur $MgSO_4$, filtrer et concentrer. Purifier le résidu par flash-chromatographie avec comme éluant un mélange : DCM/méthanol/$NH_4OH$ : 90:9:1. Le produit du titre est obtenu avec un rendement de 40 %.
$C_{15}H_{21}N_2OCl$ (PM = 280,5).
$^1$H-RMN ($CDCl_3$, 400 MHz) $\delta$ (ppm) : 0,58 (t, 3H, J = 7,2 Hz), 1,10 (t, 3H, J = 7,2 Hz), 2,08 (m, 2H), 2,91 (q, 2H, J = 7,2 Hz), 3,11 (m, 2H), 3,34 (q, 2H, J = 7,2 Hz), 3,46 (qu, 1H, J = 8,0 Hz), 7,12 (dd, 1H, J = 7,6 Hz et J = 1,2 Hz), 7,19 (m, 2H), 7,26 (m, 1H). Le signal correspondant aux H du $NH_2$ n'est pas visible sur le spectre.
SM-ESI : 281,1 ($MH^+$).

Maléate du composé du titre

La salification du composé précédent au moyen d'acide maléique permet d'obtenir le maléate du composé du titre sous la forme d'une poudre blanche.
F : 167°C.
$^1$H-RMN (DMSO $d_6$, 400 MHz) $\delta$ (ppm) : 0,50 (t, 3H, J = 6,8 Hz), 1,02 (t, 3H, J = 6,8 Hz), 2,56 (m, 2H), 2,86-2,95 (m, 4H), 3,26 (m, 2H), 3,34 (s, 1H), 3,54 (qu, 1H, J = 8,0 Hz), 6,03 (s, 2H), 7,15 (d, 1H, J = 7,6 Hz), 7,34-7,44 (m, 3H), 8,00 (s, 3H).
$^{13}$C-RMN (DMSO $d_6$, 100 MHz) $\delta$ (ppm) : 12,09, 12,12, 36,88, 39,19, 39,90, 41,14, 46,37, 123,76, 124,94, 126,61, 130,65, 133,51, 136,00, 145,09, 167,14, 170,54.
% Théorique : C 57,50, H 6,35, N 7,06.
% Trouvé : C 57,36, H 6,26, N 6,68.

Exemple 7 : *trans*-3-amino-*N,N*-diéthyl-1-(3-méthylphényl)-cyclobutanecarboxamide (1f)

Etape 1 : acide *cis*-3-hydroxy-1-(3-méthylphényl)-cyclobutanecarboxylique (B6)

Identique à l'étape 1 de l'exemple 1 en utilisant l'acide 3-méthylphénylacétique au lieu de l'acide phénylacétique. Le produit du titre est obtenu sous la forme d'un solide blanc (rendement = 40 %).
$C_{12}H_{14}O_3$ (PM = 206).
$^1$H-RMN ($CDCl_3$, 400 MHz) $\delta$ (ppm) : 2,35 (s, 3H), 2,73 (m, 2H), 2,94 (m, 2H), 4,21 (qu, 1H, J = 6,4 Hz), 7,08 (d, 1H, J = 7,6 Hz), 7,16 (s, 2H), 7,24 (m, 1H). Les signaux correspondant aux H des OH de l'alcool et de l'acide ne sont pas visibles sur le spectre.
SM-ESI : 205.

Etape 2 : 4-(3-méthylphényl)-2-oxabicyclo[2.1.1]hexane-3-one (C6)

Identique à l'étape 2 décrite dans l'exemple 1. Le produit du titre est obtenu sous la forme d'une huile incolore (rendement = 74 %).
$C_{12}H_{12}O_2$ (PM = 188).
$^1$H-RMN (DMSO $d_6$, 400 MHz) $\delta$ (ppm) : 2,37 (s, 3H), 2,70 (m, 2H), 2,87 (m, 2H), 4,96 (s, 1H), 7,09-7,30 (m, 4H).
SM-ESI : 189 ($MH^+$).

Etape 3 : *cis*-3-hydroxy-*N,N*-diéthyl-1-(3-méthylphényl)-cyclobutanecarboxamide (D6a)

Identique à l'étape 3 décrite dans l'exemple 1. Le produit du titre est obtenu avec un rendement de 77 %. $^1$H-RMN ($CDCl_3$, 400 MHz) $\delta$ (ppm) : 0,65 (t, 3H, J = 7,2 Hz), 1,08 (t, 3H, J = 7,2 Hz), 2,69 (s, 3H), 2,73 (m, 2H), 2,81 (m, 2H), 2,90 (q, 2H, J = 7,2 Hz), 3,31 (q, 2H, J = 7,2 Hz), 4,35 (qu, 1H, J = 7,4 Hz), 7, 04 (m, 1H), 7, 11 (m, 2H), 7,23 (m, 1H). Le signal correspondant à l'H du OH n'est pas visible sur le spectre.

Etape 4 : *trans*-3-azido-*N,N*-diéthyl-1-(3-méthylphényl)-cyclobutanecarboxamide (E6a)

Identique à l'étape 4 décrite dans l'exemple 1. Le produit du titre est obtenu avec un rendement de 70 %. $^1$H-RMN ($CDCl_3$, 400 MHz) $\delta$ (ppm) : 0,54 (t, 3H, J = 7,2 Hz), 1,11 (t, 3H, J = 7,2 Hz), 2,34 (s, 3H), 2,47 (m, 2H), 2,89 (q, 2H, J = 7,2 Hz), 3,12 (m, 2H), 3,34 (q, 2H, J = 7,2 Hz), 3,95 (qu, 1H, J = 7,8 Hz), 7,04 (m, 3H), 7, 2 3 (m, 1 H) .

Etape 5 : *trans*-3-amino-*N,N*-diéthyl-1-(3-méthylphényl)-cyclobutanecarboxamide (1f)

Identique à l'étape 5 décrite dans l'exemple 1. Le produit du titre est obtenu avec un rendement de 57 %. $C_{16}H_{24}N_2O$ (PM =260).
$^1$H-RMN ($CDCl_3$, 400 MHz) $\delta$ (ppm) : 0,52 (t, 3H, J = 7,2 Hz), 1,10 (t, 3H, J = 7,2 Hz), 2,11 (m, 2H), 2,33 (s, 3H), 2,92 (q, 2H, J = 7,2 Hz), 3,10 (m, 2H), 3,33 (q, 2H, J = 7,2 Hz), 3, 44 (qu, 1H, J = 8,0 Hz), 7, 03 (m, 3H), 7, 21 (m, 1H). Le signal correspondant aux H du $NH_2$ n'est pas visible sur le spectre.
SM-ESI : 261 ($MH^+$).

Maléate du composé du titre

La salification du composé précédent au moyen d'acide maléique permet d'obtenir le maléate du composé du titre sous la forme d'une poudre blanche.
F : 173°C.
$^1$H-RMN (DMSO $d_6$, 400 MHz) $\delta$ (ppm) : 0,45 (t, 3H, J = 6,8 Hz), 1,02 (t, 3H, J = 6,8 Hz), 2,31 (s, 3H), 2,52 (m, 2H), 2,89 (m, 4H), 3,25 (q, 2H, J = 6,8 Hz), 3,52 (qu, 1H, J = 8,4 Hz), 6,02 (s, 2H), 7,05 (m, 3H), 7,27 (t, 1H, J = 7,6 Hz), 8,00 (s, 2H). Le signal correspondant aux H du $NH_2$ n'est pas visible sur le spectre.
$^{13}$C-RMN (DMSO $d_6$, 100 MHz) $\delta$ (ppm) : 12,07, 12,13, 21,05, 36,97, 39,15, 40,09, 41,18, 46,56, 48,53, 121,99, 125,43, 127,15, 128,63, 136,07, 137,88, 142,66, 167,21, 171,19.
% Théorique : C 63,81, H 7,50, N 7,44.
% Trouvé : C 63,93, H 7,45, N 7,27.

Exemple 8 : *trans*-3-amino-*N,N*-diéthyl-1-(2-fluoro-3-chlorophényl)-cyclobutanecarboxamide (1g)

Etape 1 : acide *cis*-3-hydroxy-1-(2-fluoro-3-chlorophényl)-cyclobutanecarboxylique (B7)

Identique à l'étape 1 de l'exemple 1 en utilisant l'acide 2-fluoro-3-chlorophénylacétique au lieu de l'acide phénylacétique. Le produit du titre est obtenu sous la forme d'un solide blanc (rendement = 30 %).
$C_{11}H_{10}FClO_3$ (PM = 244,5).
$^1$H-RMN (DMSO $d_6$, 400 MHz) $\delta$ (ppm) : 2,58 (m, 2H), 2,75 (m, 2H), 3, 93 (qu, 1H, J = 7,6 Hz), 5, 33 (s, 1H), 7, 22 (m, 1H), 7,52 (m, 2H), 12,56 (m, 1H).
SM-ESI : 243,0.

Etape 2 : 4-(2-fluoro-3-chlorophényl)-2-oxabicyclo[2.1.1]hexane-3-one (C7)

Identique à l'étape 2 décrite dans l'exemple 1. Le produit du titre est obtenu sous la forme d'une huile incolore (rendement = 66 %).
$C_{11}H_8O_2ClF$ (PM = 226, 5) .
$^1$H-RMN (DMSO $d_6$, 400 MHz) $\delta$ (ppm) : 2,89 (s, 4H), 5,16 (s, 1H), 7,22-7,29 (m, 2H), 7,61 (m, 1H).
SM-ESI : 227 ($MH^+$).

Etape 3 : *cis*-3-hydroxy-*N,N*-diéthyl-1-(2-fluoro-3-chlorophényl)-cyclobutanecarboxamide (D7a)

Identique à l'étape 3 décrite dans l'exemple 1. Le produit du titre est obtenu avec un rendement de 87 %. $C_{15}H_{19}NO_2ClF$ (PM = 299,5).

$^1$H-RMN (DMSO $d_6$, 400 MHz) δ (ppm) : 0,35 (t, 3H, J = 7,0 Hz), 0,96 (t, 3H, J = 7,0 Hz), 2,57 (m, 2H), 2,67 (m, 2H), 2,87 (q, 2H, J = 7,0 Hz), 3,16 (q, 2H, J = 7,0 Hz), 4,00 (se, 1H, J = 8,0 Hz), 5,02 (d, 1H, J = 7,2 Hz), 7,27 (t, 1H, J = 8,0 Hz), 7,50 (t, 1H, J = 8,0 Hz), 7,65 (t, 1H, J = 8,0 Hz).
SM-ESI : 300 ($MH^+$).

Etape 4 : *trans*-3-(dioxoisoindoline-2-yl)-*N,N*-diéthyl-1-(2-fluoro-3-chlorophényl)-cyclobutanecarboxamide (F7a)

Identique à l'étape 4 décrite pour l'exemple 6. Le produit du titre est obtenu avec un rendement de 45 %. $C_{23}H_{22}FClN_2O_3$ (PM = 428, 5) .
$^1$H-RMN ($CDCl_3$, 400 MHz) δ (ppm) : 0,29 (t, 3H, J = 6,8 Hz), 1,04 (t, 3H, J = 6,8 Hz), 2,94-3,04 (m, 4H), 3,22-3,28 (m, 4H), 4,61 (qu, 1H, J = 8,8 Hz), 7.35 (t, 1H, J = 8,0 Hz), 7,54 (t, 1H, J = 8,4 Hz), 7,62 (t, 1H, J = 7,2 Hz), 7,83 (s, 4H).
SM-ESI : 429 ($MH^+$).

Etape 5 : *trans*-3-amino-*N,N*-diéthyl-1-(2-fluoro-3-chlorophényl)-cyclobutanecarboxamide (1g)

Identique à l'étape 5 décrite pour l'exemple 6. Le produit du titre est obtenu avec un rendement de 93 %. $C_{15}H_{20}FClN_2O$ (PM = 298,5).
$^1$H-RMN (DMSO $d_6$, 400 MHz) δ (ppm) : 0,27 (t, 3H, J = 6,8 Hz), 0,97 (t, 3H, J = 6,8 Hz), 2,08 (m, 2H), 2,88-2,94 (m, 4H), 3,17-3,25 (m, 3H), 7,26 (t, 1H, J = 8,0 Hz), 7,44-7,49 (m, 2H). Le signal correspondant aux H du $NH_2$ n'est pas visible sur le spectre.
SM-ESI : 299 ($MH^+$).

Maléate du composé du titre

La salification du composé précédent au moyen d'acide maléique permet d'obtenir le maléate du composé du titre sous la forme d'une poudre blanche.
F : 179°C.
$^1$H-RMN (DMSO $d_6$, 400 MHz) δ (ppm) : 0,26 (t, 3H, J = 6,8 Hz), 0,99 (t, 3H, J = 6,8 Hz), 2,60 (m, 2H), 2,91-3,00 (m, 4H), 3,20 (q, 2H, J = 6,8 Hz), 3,34 (s, 1H), 3,61 (qu, 1H, J = 8,4 Hz), 6,02 (s, 2H), 7,32 (t, 1H, J = 8,0 Hz), 7,52-7,57 (m, 2H), 7,97 (s, 3H).
$^{13}$C-RMN (DMSO $d_6$, 100 MHz) δ (ppm) : 12,15, 12,21, 36,19, 40,08, 40,56, 41,16, 43,81, 120,16, 125,59, 127,11, 129,12, 132,00, 136,11, 153,74, 156,22, 167,19, 169,48.
% Théorique : C 55,01, H 5,83, N 6,75.
% Trouvé : C 54,73, H 5,98, N 6,46.

Exemple 9 : *trans*-3-amino-*N,N*-diéthyl-1-(2,5-difluorophényl)-cyclobutanecarboxamide (1h)

Etape 1 : acide *cis*-3-hydroxy-1-(2,5-difluorophényl)-cyclobutanecarboxylique (B8)

Identique à l'étape 1 décrite dans l'exemple 1 en utilisant l'acide 2,5-difluorophénylacétique comme acide de départ. Le produit du titre est obtenu sous la forme d'un solide blanc (rendement = 69 %).
$^1$H-RMN (DMSO $d_6$, 400 MHz) δ (ppm) : 2,55 (m, 2H), 2,71 (m, 2H), 3,94 (qu, 1H, J = 7,2 Hz), 5,32 (s, 1H), 7,12-7,23 (m, 2H), 7,41 (m, 1H), 12,48 (s, 1H).

Etape 2 : 4-(2,5-difluophényl)-2-oxabicyclo[2.1.1]hexane-3-one (C8)

Identique à l'étape 2 décrite dans l'exemple 1. Le produit du titre est obtenu sous la forme d'une huile incolore (rendement = 91 %).
$C_{11}H_8F_2O_2$ (PM = 210).
$^1$H-RMN ($CDCl_3$, 400 MHz) δ (ppm) : 2,77 (m, 2H), 2,98 (m, 2H), 5,01 (s, 1H), 6,93-7,08 (m, 3H).
SM-ESI : 228 ($M+NH4^+$).

Etape 3 : *cis*-3-hydroxy-*N,N*-diéthyl-1-(2,5-difluorophényl)-cyclobutanecarboxamide (D8a)

Identique à l'étape 3 de l'exemple 1. Le produit du titre est obtenu sous la forme d'un solide blanc (rendement = 100 %).
$C_{15}H_{19}NO_2F_2$ (PM = 283).
$^1$H-RMN ($CDCl_3$, 400 MHz) δ (ppm) : 0,56 (t, 3H, J = 6,8 Hz), 1,09 (t, 3H, J = 6,8 Hz), 2,82 (m, 5H), 2,95 (q, 2H, J = 6,8

Hz), 3,31 (q, 2H, J = 6,8 Hz), 4,32 (qu, 1H, J = 7,2 Hz), 6,91-7,11 (m, 3H).
SM-ESI : 284 ($MH^+$).

Etape 4 : *trans*-3-azido-*N,N*-diéthyl-1-(2,5-difluorophényl)-cyclobutanecarboxamide (E8a)

Identique à l'étape 4 décrite dans l'exemple 1. Le produit du titre est obtenu sous la forme d'une huile incolore (rendement = 76 %).
$C_{15}H_{18}N_4OF_2$ (PM = 308).
$^1$H-RMN ($CDCl_3$, 400 MHz) δ (ppm) : 0,51 (t, 3H, J = 7,2 Hz), 1,10 (t, 3H, J = 7,2 Hz), 2,51 (m, 2H), 2,98 (q, 2H, J = 7,2 Hz), 3, 19 (m, 2H), 3, 32 (q, 2H, J = 7, 2 Hz), 4, 02 (qu, 1H, J = 8,0 Hz), 6, 90-7, 04 (m, 3H).
SM-ESI : 309 ($MH^+$).

Etape 5 : *trans*-3-amino-*N,N*-diéthyl-1-(2,5-difluorophényl)-cyclobutanecarboxamide (1h)

Introduire dans un ballon 1 eq du composé (E8a) et le dissoudre dans 20 volumes de THF. Se placer sous agitation et sous atmosphère d'azote, puis ajouter 1 volume d'eau et 1,5 eq de triphénylphosphine. Laisser ainsi sous agitation toute la nuit. Evaporer le THF sous pression réduite et reprendre le résidu obtenu par de l'eau et extraire deux fois avec du DCM. Sécher les phases organiques sur $MgSO_4$, filtrer puis évaporer le solvant sous pression réduite. L'huile obtenue est purifiée par flash-chromatographie avec comme éluant le mélange : DCM/méthanol/$NH_4OH$ 95:4,5:0,5. Le produit du titre est obtenu sous forme d'huile incolore avec un rendement de 97 %.
$C_{15}H_{20}N_2OF_2$ (PM = 282).
$^1$H-RMN ($CDCl_3$, 400 MHz) δ (ppm) : 0,51 (t, 3H, J = 6,8 Hz), 1,10 (t, 3H, J = 6,8 Hz), 2,15 (m, 2H), 2,99 (q, 2H, J = 6,8 Hz), 3,16 (m, 2H), 3, 3 2 (q, 2H, J = 6,8 Hz), 3, 5 2 (qu, 1H, J = 8,0 Hz), 6,85-7,02 (m, 3H). Le signal correspondant aux H du $NH_2$ n'est pas visible sur le spectre.
SM-ESI : 283 ($MH^+$).

Maléate du composé du titre

La salification du composé précédent au moyen d'acide maléique permet d'obtenir le maléate du composé du titre sous la forme d'une poudre blanche.
F : 184°C.
$^1$H-RMN (DMSO $d_6$, 400 MHz) δ (ppm) : 0,31 (t, 3H, J = 6,8 Hz), 0,99 (t, 3H, J = 6,8 Hz), 2,58 (m, 2H), 2,93-2,97 (m, 4H), 3,20 (q, 2H, J = 6,8 Hz), 3,34 (s, 1H), 3,59 (qu, 1H, J = 8,4 Hz), 6,02 (s, 2H), 7,13-7,30 (m, 2H), 7,49-7,53 (m, 1H), 7,97 (s, 3H).
$^{13}$C-RMN (DMSO $d_6$, 100 MHz) δ (ppm) : 12, 04, 12, 15, 36,08, 40,00, 40,61, 41,20, 43,48, 114,90, 117,2, 124,37, 132,1, 136,00, 154,6, 157,05, 157,13, 159,51, 167,12, 169,41.
% Théorique : C 57,28, H 6,07, N 7,03.
% Trouvé C : 57,21, H 6,01, N 6,66.

Exemple 10 : *trans*-3-amino-*N,N*-diéthyl-1-(3,5-dichlorophényl)-cyclobutanecarboxamide (1i)

Etape 1 : acide *cis*-3-hydroxy-1-(3,5-dichlorophényl)-cyclobutanecarboxylique (B9)

Identique à l'étape 1 décrite dans l'exemple 1 en synthétisant au préalable l'acide 3,5-dichlorophénylacétique utilisé ensuite comme acide de départ. Le produit du titre est obtenu sous la forme d'un solide blanc (rendement = 50 %).
$C_{11}H_{10}Cl_2O_3$ (PM = 261).
$^1$H-RMN (DMSO $d_6$, 400 MHz) δ (ppm) : 2,53 (m, 2H), 2,77 (m, 2H), 3,87 (qu, 1H, J = 7,4 Hz), 5,23 (s, 1H), 7,38 (m, 2H), 7,51 (m, 1H), 12,62 (s, 1H).
SM-ESI : 259.

Etape 2 : 4-(3,5-dichlorophényl)-2-oxabicyclo[2.1.1]hexane-3-one (C9)

Identique à l'étape 2 décrite dans l'exemple 1. Le produit du titre est obtenu sous la forme d'une huile incolore (rendement = 87 %).
$C_{11}H_8Cl_2O_2$ (PM = 243).
$^1$H-RMN ($CDCl_3$, 400 MHz) δ (ppm) : 2,72 (m, 2H), 2,88 (m, 2H), 4,99 (s, 1H), 7,21 (m, 2H), 7,34 (m, 1H).
SM-ESI : 244 ($MH^+$).

Etape 3 : *cis*-3-hydroxy-*N,N*-diéthyl-1-(3,5-dichlorophényl)-cyclobutanecarboxamide (D9a)

Identique à l'étape 3 de l'exemple 1. Le produit du titre est obtenu sous la forme d'un solide blanc (rendement = 100 %).
$C_{15}H_{19}Cl_2O_2N$ (PM = 316).
$^1$H-RMN ($CDCl_3$, 400 MHz) $\delta$ (ppm) : 0,77 (t, 3H, J = 7,2 Hz), 1,09 (t, 3H, J = 7,2 Hz), 2,58 (s, 1H), 2,75 (m, 4H), 2,88 (q, 2H, J = 7, 2 Hz), 3, 32 (q, 2H, J = 7, 2 Hz), 4,34 (qu, 1H, J = 7,6 Hz), 7,20-7,27 (m, 3H).
SM-ESI : 316.

Etape 4 : *trans*-3-azido-*N,N*-diéthyl-1-(3,5-dichlorophényl)-cyclobutanecarboxamide (E9a)

Identique à l'étape 4 décrite dans l'exemple 1. Le produit du titre est obtenu sous la forme d'une huile incolore (rendement = 79 %).
$C_{15}H_{18}N_4OCl_2$ (PM = 341).
$^1$H-RMN ($CDCl_3$, 400 MHz) $\delta$ (ppm) : 0,67 (t, 3H, J = 7,2 Hz), 1,12 (t, 3H, J = 7,2 Hz), 2,40 (m, 2H), 2,87 (q, 2H, J = 7,2 Hz), 3,15 (m, 2H), 3,36 (q, 2H, J = 7,2 Hz), 3,99 (qu, 1H, J = 7,6 Hz), 7,13 (m, 2H), 7,25 (m, 1H).
SM-ESI : 341.

Etape 5 : *trans*-3-amino-*N,N*-diéthyl-1-(3,5-dichlorophényl)-cyclobutanecarboxamide (1i)

Identique à l'étape 5 de l'exemple 9. Le produit du titre est obtenu sous forme d'huile incolore avec un rendement de 78 %.
$C_{15}H_{20}N_2OClF$ (PM = 283).
$^1$H-RMN (DMSO $d_6$, 400 MHz) $\delta$ (ppm) : 0,58 (t, 3H, J = 7,2 Hz), 1,00 (t, 3H, J = 7,2 Hz), 1,90 (s, 2H), 2,07 (m, 2H), 2,85 (m, 4H), 3,11 (qu, 1H, J = 8,0 Hz), 3,25 (q, 2H, J = 7,2 Hz), 7,23 (m, 2H), 7,48 (m, 1H).
SM-ESI : 283.

Maléate du composé du titre

La salification du composé précédent au moyen d'acide maléique permet d'obtenir le maléate du composé du titre sous la forme d'une poudre blanche.
F : 180°C.
$^1$H-RMN (DMSO $d_6$, 400 MHz) $\delta$ (ppm) : 0,57 (t, 3H, J = 6,8 Hz), 1,02 (t, 3H, J = 6,8 Hz), 2,58 (m, 2H), 2,85-2,96 (m, 4H), 3,28 (q, 2H, J = 6,8 Hz), 3,54 (qu, 1H, J = 8,4 Hz), 6,02 (s, 2H), 7,28 (s, 2H), 7,56 (s, 1H), 7,99 (s, 3H).
$^{13}$C-RMN (DMSO $d_6$, 100 MHz) $\delta$ (ppm) : 11, 96, 12,19, 36,83, 39,20, 41,10, 46,2, 124,03, 126,38, 134,50, 136,02, 146,66, 167,12, 170,04.
% Théorique : C 52,91, H 5,61, N 6,50.
% Trouvé : C 53,01, H 5,53, N 6,11.
Les exemples suivants permettent de mieux comprendre l'invention sans en limiter la portée.
Les composés de formule générale (1) ainsi que leurs sels pharmaceutiquement acceptables présentent des propriétés pharmacologiques remarquables : ils sont en général plus puissants que la kétamine en tant que bloqueur de canal NMDA tout en présentant moins d'effets indésirables sur le système nerveux central que la kétamine.
Nous avons examiné les effets des composés de l'invention sur l'inhibition du courant NMDA dans des ovocytes de Xénope (*Xenopus laevis*) exprimant des récepteurs NMDA humains recombinants construits à partir des sous-unités NR1 et NR2B. Les courants produits par stimulation de ces récepteurs au moyen des agonistes endogènes ont été étudiés selon la technique de "voltage-clamp à deux électrodes" rapportée par Planells-Cases et al., 2002, J. Pharmacol. Exp. Ther., 302, 163-173.
Protocole : les ovocytes sont prélevés chirurgicalement sur des Xénopes adultes, défolliculés enzymatiquement et conservés à 17°C dans une solution contenant : 96 mM de NaCl, 2 mM de KCl, 1 mM de $MgCl_2$, 1,8 mM de $CaCl_2$ et 5 mM de HEPES à pH 7,5 (NaOH) et 50 mg/L de gentamicine (Heusler et al., 2005, Neuropharmacology, 49, 963-976). L'ADN complémentaire (ADNc) codant pour la sous-unité NR1 a été cloné par PCR en utilisant des "primers" ciblés sur les codons "début" et "fin" de la séquence publiée (numéro d'accession GenBank M_007327). L'ADNc codant pour la sous-unité NR2B a été synthétisé par Eurogentec (Seraing, Belgique) selon la séquence publiée (numéro d'accession GenBank NM_000834). Les ADNc NR1 et NR2B ont ensuite été sous-clonés dans le vecteur à haute expression pGEMHE pour transcription *in vitro* de l'ADNc. Les ARNc codant pour NR1 and NR2B ont été préparés selon la méthode décrite par Heusler et *al.* (déjà cité). Les aliquotes de la solution d'ARNc ont été injectés dans les ovocytes (20-500 pg/ovocyte pour NR1 et 40-1000 pg/ovocyte pour NR2B). Chaque ovocyte est injecté avec 100 nL d'une solution contenant : 4 mM de $Na^+$BAPTA (pH 7,2) afin de bloquer tous les courants chlore résiduels. Après stabilisation, les

courants NMDA sont activés par superfusion de glutamate et de glycine chacun à la concentration de 10 $\mu$M. Les composés à tester sont ensuite superfusés dans une solution de Ringer $Ba^{++}$ à des concentrations croissantes en présence de glutamate et de glycine (4 à 5 concentrations sont testées par ovocyte). Les courbes concentration-réponse obtenues sont analysées pour chaque ovocyte par régression non-linéaire et une valeur de $pCI_{50}$ calculée. Par $pCI_{50}$ on entend le logarithme négatif de la concentration du composé testé nécessaire pour diminuer l'amplitude du courant NMDA de moitié.

Résultats : le tableau 1 ci-dessous donne les valeurs des $pCI_{50}$ pour certains des composés de l'invention. Il ressort que, dans les conditions du test, les composés (1a1), (1b), (1c), (1d) et (1e) bloquent le courant NMDA de façon concentration-dépendante et sont plus puissants que la kétamine, un antagoniste NMDA utilisé en clinique.

Tableau 1.

| Composé | Inhibition du courant NMDA $pCI_{50}$ |
|---|---|
| 1a1 | 6,3 |
| 1b | 6,3 |
| 1c | 6,8 |
| 1d | 6,4 |
| 1e | 7,1 |
| kétamine | 6,1 |

Compte tenu de la faible biodisponibilité par voie orale de la kétamine, nous avons choisi la voie intrapéritonéale (ip) comme voie d'administration unique dans les expériences *in vivo.* L'activité analgésique des composés de formule (1) et de la kétamine, choisie comme composé de référence, a été déterminée dans un modèle de douleur inflammatoire aiguë classique, l'injection intradermique de formaldéhyde (Bardin et al., 2001, Eur. J. Pharmacol., 421, 109-114).

Protocole : les rats mâles (Sprague-Dawley Iffa Credo, France) sont placés dans des boîtes d'observation en plexiglas au-dessus d'un miroir incliné de façon à faciliter l'observation de leurs pattes postérieures. Après 30 min d'acclimatation, les animaux reçoivent une injection de formaldéhyde dilué à 2,5 % au niveau de la surface plantaire de la patte postérieure droite. L'injection de formaldéhyde produit des réponses comportementales qui apparaissent en deux phases :

- une phase précoce, de 0 à 5 min après l'injection de formaldéhyde, correspondant à la stimulation des récepteurs spécialisés dans la transmission des stimuli nociceptifs ;
- une phase tardive qui survient entre 20 et 30 min après l'injection. Cette phase correspond à la stimulation de récepteurs par des médiateurs inflammatoires et/ou à l'hyperexcitabilité des neurones de la corne dorsale de la moelle épinière. Cette phase plus tardive met donc en jeu une sensibilisation centrale du système de neurotransmission de la douleur, dans laquelle le système glutamate/NMDA joue un rôle prépondérant. De ce fait, la douleur présente durant cette seconde phase est plus représentative des douleurs neuropathiques que celle qui survient lors de la première phase. Pour cette raison, seuls les résultats obtenus dans cette phase tardive sont pris en considération dans la présente application.

Nous avons sélectionné le léchage de la patte qui a reçu l'injection comme paramètre comportemental de quantification de la douleur et choisi comme périodes d'observation celle qui correspond à la phase tardive (c'est-à-dire 22,5-27,5 min post-injection de formaldéhyde). Durant cette phase de 5 min, les animaux sont observés toutes les 30 s afin de noter si l'animal se lèche ou non la patte "injectée" ; ainsi, le score maximum est de 10. Les produits de l'invention ou le véhicule sont administrés par voie ip 15 min avant l'injection de formaldéhyde.

Résultats : dans ce test, les composés de formules (1a1) et (1e), représentatifs des composés de l'invention, exercent une activité analgésique remarquable (Tableau 2). Ainsi, la dose minimale significative (DMS, dose nécessaire pour réduire significativement le léchage de la patte injectée) obtenue avec les composés de formules (1a1) et (1e) est inférieure à celle obtenue avec la kétamine. Un autre avantage des composés de formules (1a1) et (1e) par rapport à la kétamine se situe aussi au niveau de l'amplitude de l'effet analgésique. En effet, nous constatons qu'à la dose de 40 mg/kg, le léchage de la patte est complètement inhibé avec les composés (1a1) et (1e) alors qu'il n'atteint que 75 % de réduction avec la kétamine. Les composés (1a1) et (1e) sont donc plus puissants et plus efficaces que la kétamine.

Tableau 2.

| Composé | Léchage de la patte | |
|---|---|---|
| | DMS (mg/kg) | % réduction à 40 mg/kg |
| 1a1 | 10 | 100 |
| 1e | 10 | 100 |
| kétamine | 40 | 75 |

En résumé, l'activité analgésique des composés (1a1) et (1e), représentatifs des composés de formule (1), surpasse celle de la kétamine dans un modèle de douleur aigüe inflammatoire chez le rat.

Nous démontrons aussi que les composés de l'invention ont une activité antidépressive *in vivo.* L'activité antidépressive des composés de formule (1) et de la kétamine a été déterminée dans le modèle de la nage forcée chez le rat, modèle largement utilisé car prédictif d'une activité antidépressive chez l'homme.

Protocole : les rats mâles (Sprague-Dawley Iffa Credo, France) sont placés dans un cylindre (45 cm de hauteur et 20 cm de diamètre) rempli d'eau à 25°C $\pm$ 0,5°C jusqu'à 17 cm de hauteur. Cette hauteur permet aux rats de nager ou de flotter sans que leurs pattes ne touchent le fond du cylindre. Vingt-quatre heures avant le jour test, les rats sont placés dans le cylindre pendant 15 min, laps de temps au bout duquel ils ne tentent plus de s'échapper et restent immobiles à la surface. Le jour du test, le composé à tester ou le véhicule est injecté (ip) à l'animal qui est placé dans le cylindre 30 min plus tard. La durée d'immobilité (définie lorsque le rat se laisse flotter et qu'il effectue uniquement des petits mouvements pour se maintenir à la surface) est mesurée avec une précision de 0,1 s pendant 5 min.

Résultats : dans le test de la nage forcée, les composés de formules (1c) et (1e), représentatifs de la série, réduisent de façon significative le temps d'immobilité de l'animal. Lorsque l'on compare les $DE_{50}$, c'est-à-dire les doses qui diminuent le temps d'immobilité de moitié par rapport aux animaux témoins, on constate qu'elles sont inférieures à celle de la kétamine pour les composés (1c) et (1e), cf. Tableau 3. De même, l'amplitude de l'effet anti-immobilité observée à la dose de 20 mg/kg est supérieure avec les composés (1c) et (1e) que celle obtenue avec la kétamine.

Tableau 3.

| Composé | Temps d'immobilité | |
|---|---|---|
| | $DE_{50}$ (mg/kg) | % réduction à 20 mg/kg |
| 1c | 13 | 83 |
| 1e | 15 | 77 |
| kétamine | 20 | 50 |

En résumé, les composés (1c) et (1e), représentatifs des composés de formule (1), sont plus puissants et plus efficaces que la kétamine dans un test prédictif d'une activité antidépressive.

Nous avons déjà souligné l'importance de normaliser la fonction du récepteur NMDA, c'est-à-dire bloquer son activité excessive sans interférer ou en interférant le moins possible avec son fonctionnement physiologique normal. Comme marqueur d'interaction des produits de l'invention avec le fonctionnement normal du récepteur NMDA, nous avons choisi le test de l'inhibition pré-pulse du réflexe de sursaut (PPI). Ce test représente une mesure de la capacité de l'organisme à filtrer les informations non essentielles. Les antagonistes non compétitifs, compétitifs ainsi que les bloqueurs du canal diminuent ce phénomène de PPI chez le rat (Depoortere et al., 1999, Behav. Pharmacol., 10, 51-62), une telle diminution étant considérée comme prédictive des effets psychotomimétiques des antagonistes NMDA chez l'homme.

Protocole : les rats mâles (Sprague-Dawley Iffa Credo, Les Oncins, France) sont placés dans des cylindres de 18,4 cm de long et 8,8 cm de diamètre reposant sur une base en dessous de laquelle est fixé un accéléromètre piézoélectrique servant à détecter la réponse de sursaut. Le tout est enfermé dans une boîte possédant un haut-parleur fixé au plafond pour délivrer les pulses et pré-pulses sonores, et isolée acoustiquement (SR LAB, San Diego Instruments, San Diego, USA). Tous les événements sont contrôlés à l'aide d'un logiciel dédié. Les animaux sont d'abord soumis à un pré-test de 13 min destiné à les habituer à la procédure et à éliminer ceux ne répondant pas à une série de critères de réponse minimale. Trois types de stimuli sonores (bruit blanc) sont délivrés : 1) un pulse de 118 dB (P, durée 40 msec) ; 2) un pré-pulse de 78 dB (durée 20 msec), suivi d'un pulse de 118 dB (pP) ; et 3) pas de pré-pulse ou de pulse (NP). L'intervalle entre le début du pré-pulse et le début du pulse est de 100 msec, le bruit de fond étant de 70 dB. La réponse de sursaut est enregistrée durant 100 ms, 100 ms après le début du stimulus (pP ou NP) par une carte d'acquisition numérique/analogique de 12 bits. La session démarre avec une période sans stimulus de 5 min, à la suite

de quoi les animaux sont exposés à 10 P (séparés en moyenne par 15 s et destinés à stabiliser la réponse de sursaut). Les réponses enregistrées avec ces 10 P ne sont pas utilisées pour les calculs. Par la suite, 10 P, 10 pP et 3 NP sont délivrés dans un ordre pseudo-aléatoire espacés en moyenne de 15 s. A la fin de ce pré-test, les rats subissent une injection ip avec les composés à tester ou du sérum physiologique comme contrôle, et sont replacés dans leur boîte d'hébergement. La session de test proprement dite (en tout point similaire au pré-test) est effectuée 60 min plus tard. Le pourcentage d'inhibition du pré-pulse est calculé à l'aide des données de cette session de test, selon la formule :

(amplitude médiane P - amplitude médiane pP)

x 100/(amplitude médiane P).

Résultats : d'après la figure 1 annexée, il apparaît que le composé (1a1) ne perturbe l'inhibition du réflexe de sursaut induite par le pré-pulse (PPI) qu'à partir de la dose de 20 mg/kg. Cependant, de façon surprenante, la diminution du PPI est nettement moins marquée que celle observée avec la kétamine. En effet, à la dose de 20 mg/kg ip, la kétamine induit une disparition totale du PPI alors que le composé (1a1) ne provoque qu'une diminution de l'ordre de 30 %. La réduction du PPI reste d'ailleurs modeste même à la dose de 40 mg/kg. Par conséquent, le composé (1a1) a une tendance nettement moins marquée que la kétamine à causer des effets secondaires d'origine centrale.

En résumé, les composés de l'invention possèdent une activité analgésique et antidépressive supérieure à celle de la kétamine dans les modèles animaux décrits ci-dessus. De façon surprenante, les composés de l'invention ne provoquent des effets centraux que très modérés. Il ressort donc de ces expériences que le rapport bénéfice/risque des composés de l'invention est nettement plus favorable que celui de la kétamine. De ce fait, les composés de la présente invention ainsi que les compositions pharmaceutiques contenant comme principe actif un composé de formule générale (1) ou un de ses sels pharmaceutiquement acceptables sont potentiellement utiles comme médicaments, en particulier dans le traitement de certaines pathologies telles que, par exemple, la dépression et les douleurs, notamment les douleurs aiguës et chroniques, domaines pour lesquels les besoins thérapeutiques ne sont pas entièrement satisfaits et pour lesquels la découverte de nouveaux traitements est donc fortement souhaitable.

## Revendications

**1.** Composé de formule générale (1) suivante :

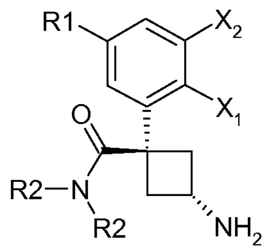

(1)

ou un sel ou solvate pharmaceutiquement acceptable de celui-ci,
pour laquelle :

- $X_1$ représente un atome d'hydrogène ou un atome de fluor ;
- $X_2$ est un atome d'hydrogène ou un atome de fluor ou un atome de chlore ;
- R1 représente un atome d'hydrogène ou un atome de fluor ou un atome de chlore ou un groupe méthyle ou un groupe méthoxy ou un groupe cyano ;
- R2 représente ensemble ou séparément un groupe méthyle ou un groupe éthyle.

**2.** Composé selon la revendication 1, **caractérisé en ce que** :

- $X_1$ représente un atome d'hydrogène ou un atome de fluor ;
- $X_2$ est un atome d'hydrogène ou un atome de fluor ou un atome de chlore ;
- R1 représente un atome d'hydrogène ou un atome de fluor ou un atome de chlore ou un groupe méthyle ou un groupe méthoxy ou un groupe cyano ;
- R2 est un groupe éthyle.

**3.** Composé selon l'une quelconque des revendications 1 ou 2, **caractérisé en ce qu'**il est choisi parmi les composés suivants :

- *trans*-3-amino-*N,N*-diéthyl-1-phénylcyclobutanecarboxamide,
- *trans*-3-amino-*N,N*-diméthyl-1-phénylcyclobutanecarboxamide,
- *trans*-3-amino-*N,N*-diéthyl-1-(2-fluorophényl)-cyclobutanecarboxamide,
- *trans*-3-amino-*N,N*-diéthyl-1-(3-méthoxyphényl)-cyclobutanecarboxamide,
- *trans*-3-amino-*N,N*-diéthyl-1-(3-fluorophényl)-cyclobutanecarboxamide,
- *trans*-3-amino-*N,N*-diéthyl-1-(3-chlorophényl)-cyclobutanecarboxamide,
- *trans*-3-amino-*N,N*-diéthyl-1-(3-méthylphényl)-cyclobutanecarboxamide,
- *trans*-3-amino-*N,N*-diéthyl-1-(3-cyanophényl)-cyclobutanecarboxamide,
- *trans*-3-amino-*N,N*-diéthyl-1-(2-fluoro-3-chlorophényl)-cyclobutanecarboxamide,
- *trans*-3-amino-*N,N*-diéthyl-1-(2,5-difluorophényl)-cyclobutanecarboxamide,
- *trans*-3-amino-*N,N*-diéthyl-1-(3,5-difluorophényl)-cyclobutanecarboxamide,
- *trans*-3-amino-*N,N*-diéthyl-1-(3,5-dichlorophényl)-cyclobutanecarboxamide.

**4.** Composé selon l'une quelconque des revendications 1 à 3, pour son utilisation en tant que médicament.

**5.** Composé selon l'une quelconque des revendications 1 à 3, pour son utilisation en tant que médicament destiné au traitement de la dépression.

**6.** Composé selon l'une quelconque des revendications 1 à 3, pour son utilisation en tant que médicament destiné au traitement de la douleur, notamment des douleurs par excès de nociception, des douleurs neuropathiques et des douleurs mixtes.

**7.** Composition pharmaceutique comprenant au moins un composé de formule générale (1) selon l'une quelconque des revendications 1 à 3, et au moins un excipient pharmaceutiquement acceptable.

**8.** Composition pharmaceutique, selon la revendication 7, pour son utilisation en tant que médicament destiné au traitement et/ou à la prévention de la dépression.

**9.** Composition pharmaceutique, selon la revendication 7, pour son utilisation en tant que médicament destiné au traitement de la douleur, notamment les douleurs par excès de nociception, les douleurs neuropathiques et mixtes.

**10.** Composition pharmaceutique, selon l'une quelconque des revendications 7 à 9, **caractérisée en ce qu'**elle est formulée pour une administration orale.

**11.** Composition pharmaceutique, selon l'une quelconque des revendications 7 à 9, **caractérisée en ce qu'**elle est formulée pour une administration topique.

**12.** Composition pharmaceutique selon l'une quelconque des revendications 8 à 11, **caractérisée en ce qu'**elle est présentée sous la forme d'une unité de dosage quotidienne d'un composé de formule générale (1) comprise entre 1 et 1000 mg.

**13.** Procédé de préparation des composés de formule générale(1) tels que définis à l'une des revendications 1 à 3, **caractérisé en ce que** l'on fait réagir une amine secondaire de formule $(R2)_2NH$ sur un composé de formule (C)

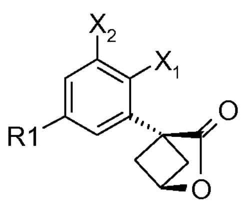

(C)

pour donner le composé de formule (D)

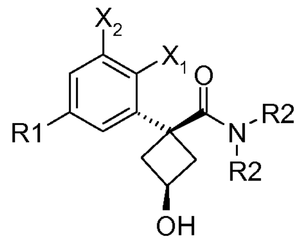

(D)

le composé de formule (D) est ensuite converti en l'amine de formule (1), les radicaux R1, R2, $X_1$ et $X_2$ présents dans les réactifs ci-dessus étant tels que définis dans la revendication 1.

**14.** Intermédiaires de synthèse de formule (D)

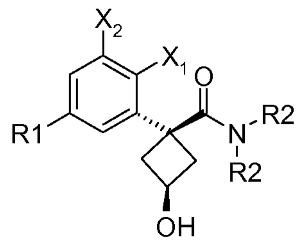

(D)

dans lequel R1, R2, $X_1$ et $X_2$ sont tels que définis dans la revendication 1, utilisés pour la préparation des composés de formule générale (1) telle que définie à l'une des revendications 1 à 3.

**15.** Intermédiaire de synthèse de formule (C)

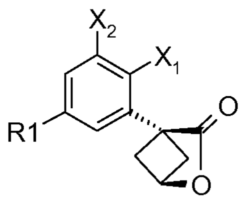

(C)

dans lequel R1, $X_1$ et $X_2$ sont tels que définis dans la revendication 1, utilisés pour la préparation des composés de formule générale (D) telle que définie à la revendication 14.

**Patentansprüche**

**1.** Verbindung der folgenden allgemeinen Formel (1):

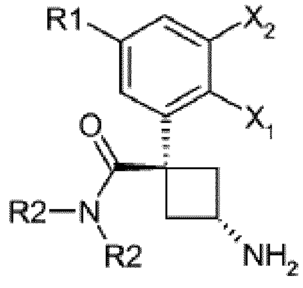

(1)

oder ein pharmazeutisch akzeptables Salz oder Solvat davon,
in der:

- $X_1$ für ein Wasserstoffatom oder ein Fluoratom steht;
- $X_2$ ein Wasserstoffatom oder ein Fluoratom oder ein Chloratom ist;
- R1 für ein Wasserstoffatom oder ein Fluoratom oder ein Chloratom oder eine Methylgruppe oder eine Methoxygruppe oder eine Cyanogruppe steht;
- R2 gemeinsam oder einzeln für eine Methylgruppe oder eine Ethylgruppe steht.

**2.** Verbindung nach Anspruch 1, **dadurch gekennzeichnet, dass**:

- $X_1$ für ein Wasserstoffatom oder ein Fluoratom steht;
- $X_2$ ein Wasserstoffatom oder ein Fluoratom oder ein Chloratom ist;
- R1 für ein Wasserstoffatom oder ein Fluoratom oder ein Chloratom oder eine Methylgruppe oder eine Methoxygruppe oder eine Cyanogruppe steht;
- R2 eine Ethylgruppe ist.

**3.** Verbindung nach einem der Ansprüche 1 oder 2, **dadurch gekennzeichnet, dass** sie aus den folgenden Verbindungen ausgewählt ist:

- *trans*-3-Amino-*N,N*-diethyl-1-phenylcyclobutancarboxamid,
- *trans*-3-Amino-*N,N*-dimethyl-1-phenylcyclobutancarboxamid,
- *trans*-3-Amino-*N,N*-diethyl-1-(2-fluorphenyl)cyclobutancarboxamid,
- *trans*-3-Amino-*N,N*-diethyl-1-(3-methoxyphenyl)cyclobutancarboxamid,
- *trans*-3-Amino-*N,N*-diethyl-1-(3-fluorphenyl)cyclobutancarboxamid,
- *trans*-3-Amino-*N,N*-diethyl-1-(3-chlorphenyl)cyclobutancarboxamid,
- *trans*-3-Amino-*N,N*-diethyl-1-(3-methylphenyl)cyclobutancarboxamid,
- *trans*-3-Amino-*N,N*-diethyl-1-(3-cyanophenyl)cyclobutancarboxamid,
- *trans*-3-Amino-*N,N*-diethyl-1-(2-fluor-3-chlorphenyl)cyclobutancarboxamid,
- *trans*-3-Amino-*N,N*-diethyl-1-(2,5-difluorphenyl)cyclobutancarboxamid,
- *trans*-3-Amino-*N,N*-diethyl-1-(3,5-difluorphenyl)cyclobutancarboxamid,
- *trans*-3-Amino-*N,N*-diethyl-1-(3,5-dichlorphenyl)cyclobutancarboxamid.

**4.** Verbindung nach einem der Ansprüche 1 bis 3 zu deren Verwendung als Arzneimittel.

**5.** Verbindung nach einem der Ansprüche 1 bis 3 zu deren Verwendung als Arzneimittel, das zur Behandlung von Depression vorgesehen ist.

**6.** Verbindung nach einem der Ansprüche 1 bis 3 zu deren Verwendung als Arzneimittel, das zur Behandlung von Schmerzen vorgesehen ist, insbesondere Schmerzen aufgrund von exzessiver Nozizeption, neuropathische Schmerzen und gemischte Schmerzen.

**7.** Pharmazeutische Zusammensetzung, die mindestens eine Verbindung der allgemeinen Formel (1) nach einem der Ansprüche 1 bis 3 und mindestens einen pharmazeutisch akzeptablen Hilfsstoff umfasst.

**8.** Pharmazeutische Zusammensetzung nach Anspruch 7 zu deren Verwendung als Arzneimittel, das zur Behandlung und/oder Verhinderung von Depression vorgesehen ist.

**9.** Pharmazeutische Zusammensetzung nach Anspruch 7 zu deren Verwendung als Arzneimittel, das zur Behandlung von Schmerzen vorgesehen ist, insbesondere Schmerzen aufgrund von exzessiver Nozizeption, neuropathische und gemischte Schmerzen.

**10.** Pharmazeutische Zusammensetzung nach einem der Ansprüche 7 bis 9, **dadurch gekennzeichnet, dass** sie für eine orale Verabreichung formuliert wird.

**11.** Pharmazeutische Zusammensetzung nach einem der Ansprüche 7 bis 9, **dadurch gekennzeichnet, dass** sie für eine topische Verabreichung formuliert wird.

**12.** Pharmazeutische Zusammensetzung nach einem der Ansprüche 8 bis 11, **dadurch gekennzeichnet, dass** sie in der Form einer Tageseinheitsdosis einer Verbindung der allgemeinen Formel (1) vorliegt, die zwischen 1 und 1000 mg liegt.

**13.** Verfahren zur Herstellung von Verbindungen der allgemeinen Formel (1), wie in einem der Ansprüche 1 bis 3 definiert, **dadurch gekennzeichnet, dass** ein sekundäres Amin der Formel $(R2)_2NH$ mit einer Verbindung der Formel (C)

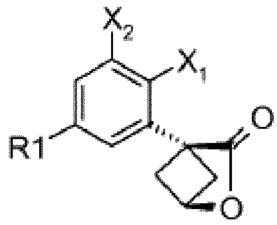

(C)

umgesetzt wird, um die Verbindung der Formel (D)

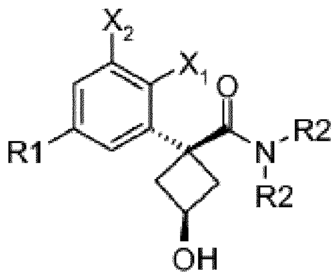

(D)

zu ergeben, wobei die Verbindung der Formel (D) anschließend in das Amin der Formel (1) umgewandelt wird, wobei die Reste R1, R2, $X_1$ und $X_2$, die in den obigen Reaktanten vorliegen, wie in Anspruch 1 definiert sind.

**14.** Synthesezwischenverbindungen der Formel (D)

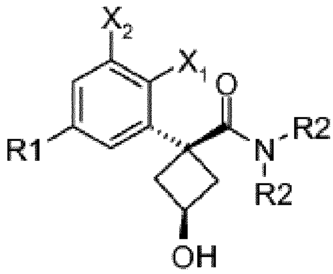

(D) ,

wobei R1, R2, $X_1$ und $X_2$ wie in Anspruch 1 definiert sind, die zur Herstellung von Verbindungen der allgemeinen Formel (1), wie in einem der Ansprüche 1 bis 3 definiert, verwendet werden.

**15.** Synthesezwischenverbindung der Formel (C)

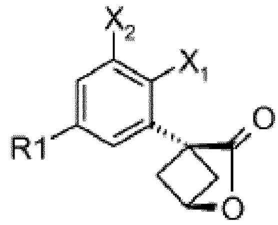

(C) ,

wobei R1, $X_1$ und $X_2$ wie in Anspruch 1 definiert sind, die zur Herstellung von Verbindungen der allgemeinen Formel (D), wie in Anspruch 14 definiert, verwendet wird.

## Claims

**1.** Compound of the following general formula (1):

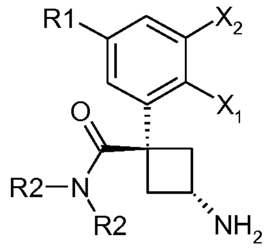

(1)

or pharmaceutically acceptable salt or solvate thereof,
wherein:

- $X_1$ represents a hydrogen atom or fluorine atom;
- $X_2$ is a hydrogen atom or fluorine atom or chlorine atom;
- R1 represents a hydrogen atom or fluorine atom or chlorine atom or methyl group or methoxy group or cyano group;
- R2 represents independently or together a methyl group or ethyl group.

**2.** Compound according to claim 1, **characterised in that**:

- $X_1$ represents a hydrogen atom or fluorine atom;
- $X_2$ is a hydrogen atom or fluorine atom or chlorine atom;
- R1 a hydrogen atom or fluorine atom or chlorine atom or methyl group or methoxy group or cyano group;
- R2 is an ethyl group.

**3.** Compound according to any one of claims 1 or 2, **characterised in that** it is chosen from among the following compounds:

- *trans*-3-amino-*N,N*-diethyl-1-phenylcyclobutanecarboxamide,
- *trans*-3-amino-*N,N*-dimethyl-1-phenylcyclobutanecarboxamide
- *trans*-3-amino-*N,N*-diethyl-1-(2-fluorophenyl)-cyclobutanecarboxamide,
- *trans*-3-amino-*N,N*-diethyl-1-(3-methoxyphenyl)-cyclobutanecarboxamide,
- *trans*-3-amino-*N,N*-diethyl-1-(3-fluorophenyl)-cyclobutanecarboxamide,
- *trans*-3-amino-*N,N*-diethyl-1-(3-chlorophenyl)-cyclobutanecarboxamide,
- *trans*-3-amino-*N,N*-diethyl-1-(3-methylphenyl)-cyclobutanecarboxamide,
- *trans*-3-amino-*N,N*-diethyl-1-(3-cyanophenyl)-cyclobutanecarboxamide,
- *trans*-3-amino-*N,N*-diethyl-1-(2-fluoro-3-chlorophenyl)-cyclobutanecarboxamide,
- *trans*-3-amino-*N,N*-diethyl-1-(2,5-difluorophenyl)-cyclobutanecarboxamide,
- *trans*-3-amino-*N,N*-diethyl-1-(3,5-difluorophenyl)-cyclobutanecarboxamide,
- *trans*-3-amino-*N,N*-diethyl-1-(3,5-dichlorophenyl)-cyclobutanecarboxamide.

**4.** Compound according to any one of claims 1 to 3 for use as a medication.

**5.** Compound according to any one of claims 1 to 3 for use as medication for the treatment of depression.

**6.** Compound according to any one of claims 1 to 3 for use as medication for the treatment of pain, particularly pain due to excessive nociception, neuropathic pain and mixed pain.

**7.** Pharmaceutical composition comprising at least one compound of general formula (1) according to any one of claims 1 to 3, and at least once pharmaceutically acceptable excipient.

**8.** Pharmaceutical composition according to claim 7 for use as medication for the treatment and/or prevention of depression.

**9.** Pharmaceutical composition according to claim 7 for use as medication for the treatment of pain, particularly pain due to excessive nociception, neuropathic pain and mixed pain.

**10.** Pharmaceutical composition according to any one of claims 7 to 9, **characterised in that** it is formulated for oral administration.

**11.** Pharmaceutical composition according to any one of claims 7 to 9, **characterised in that** it is formulated for topical administration.

**12.** Pharmaceutical composition according to any one of claims 8 to 11, **characterised in that** it is presented in the form of a daily dosage unit of a compound of general formula (1) between 1 and 1000 mg.

**13.** Method for the preparation of compounds of general formula (1) as defined in one of claims 1 to 3, **characterised in that** a secondary amine of formula $(R2)_2NH$ is reacted with a compound of formula (C)

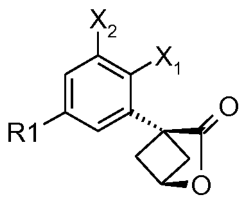

(C)

to give the compound of formula (D)

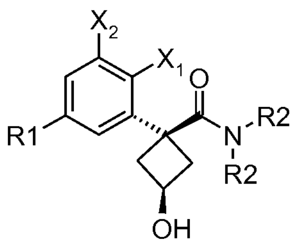

(D)

the compound of formula (D) is then converted into an amine of formula (1), the R1, R2, $X_1$ and $X_2$ radicals present in the reagents hereinabove being as defined in claim 1.

**14.** Synthesis intermediates of formula (D)

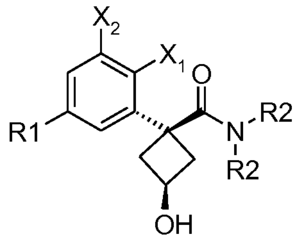

(D)

wherein R1, R2, $X_1$ and $X_2$ ae as defined in claim 1, used for the preparation of compounds of general formula (1) such as defined in claims 1 to 3.

**15.** Synthesis intermediates of formula (C)

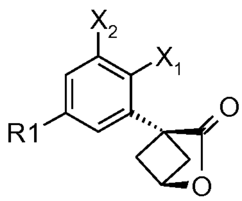

(C)

wherein R1, $X_1$ and $X_2$ are as defined in claim 1, used for the preparation of compounds of general formula (D) such as defined in claim 14.

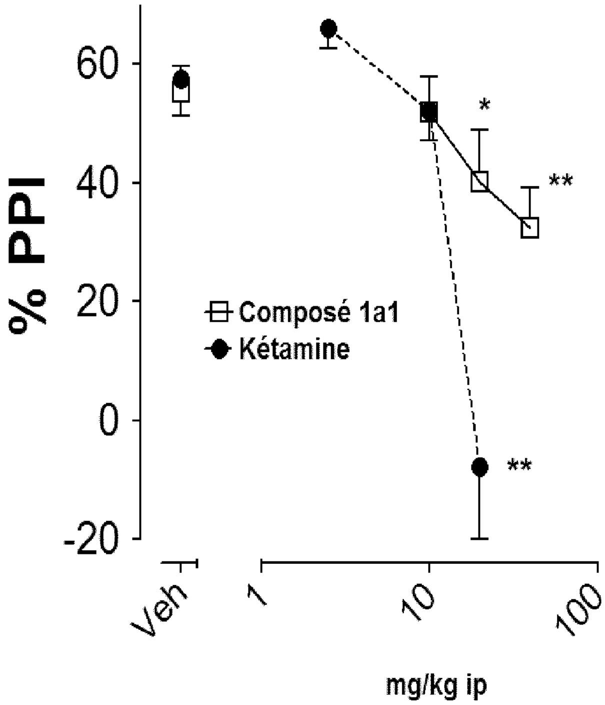


Figure 1

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- US 6387957 B
- WO 03061656 A
- WO 9807447 A
- WO 9912537 A
- US 20040204366 A
- WO 2010036937 A
- US 20040101582 A
- WO 2000003716 A
- WO 2009029618 A
- WO 2010037533 A
- US 2011160260 A
- WO 2009092324 A
- WO 2009069610 A
- WO 2010074647 A
- WO 2010142890 A
- EP 0747348 A
- WO 2003063797 A
- WO 9952848 A
- WO 2010112597 A
- WO 2000051607 A
- WO 2007038452 A
- WO 2008092955 A
- WO 2006081179 A


### Littérature non-brevet citée dans la description


- **KARADOTTIR et al.** *Nature,* 2005, vol. 438, 1162-1166
- **CHEN ; LIPTON.** *J. Neurochem.,* 2006, vol. 97, 1611-1626
- **ZARATE et al.** *Arch. Gen. Psychiatry,* 2006, vol. 63, 856-864
- **JOHANNES et al.** *J. Pain,* 2010, vol. 11, 1230-1239
- **COHEN et al.** *Adv. Psychosom. Med.,* 2011, vol. 30, 139-161
- **FINCH et al.** *Pain,* 2009, vol. 146, 18-25
- **ELIA ; TRAMER.** *Pain,* 2005, vol. 113, 61-70
- **MUIR.** *Current Opinion in Pharmacology,* 2006, vol. 6, 53-60
- **WILDER-SMITH et al.** *Pain,* 2002, vol. 97, 189-194
- **BELOEIL et al.** *Anesth. Analg.,* 2009, vol. 109, 943-950
- **SALTER et al.** *Biorheology,* 2004, vol. 41, 273-281
- **PIEPOLI et al.** *Osteoarthritis and Cartilage,* 2009, vol. 17, 1076-1083
- **AARTS ; TYMIANSKI.** *Biochem. Pharmacol.,* 2003, vol. 66, 877-886
- **WALLACE et al.** *Neurology,* 2002, vol. 59, 1694-1700
- **MONY et al.** *Br. J. Pharmacol.,* 2009, vol. 157, 1301-1317
- **ZARANTONELLO et al.** *Bioorg. Med. Chem. Lett.,* 2011, vol. 21, 2059-2063
- Remington, The science and Practice of Pharmacy. Mack Publishing Company, 1995
- **WILLIAMS et al.** *Tetrahedron Lett.,* 1995, vol. 36, 5461-5464
- **SOLTANI RAD et al.** *Tetrahedron Lett.,* 2007, vol. 48, 3445-3449
- **PLANELLS-CASES et al.** *J. Pharmacol. Exp. Ther.,* 2002, vol. 302, 163-173
- **HEUSLER et al.** *Neuropharmacology,* 2005, vol. 49, 963-976
- **BARDIN et al.** *Eur. J. Pharmacol,* 2001, vol. 421, 109-114
- **DEPOORTERE et al.** *Behav. Pharmacol.,* 1999, vol. 10, 51-62